# EUROPEAN PATENT SPECIFICATION

(11) **EP 4 117 562 B1**
(45) Date of publication and mention of the grant of the patent: **21.05.2025**
(21) Application number: 21717621.3
(22) Date of filing: 12.03.2021
(51) Int. Cl.: A61B 18/18

(54) **RAMPING UP FUNCTION FOR MICROWAVE ABLATION DEVICES**
STEIGERUNGSFUNKTION FÜR MIKROWELLENABLATIONSVORRICHTUNGEN
FONCTION DE MONTÉE EN CHARGE POUR DISPOSITIFS D'ABLATION À MICRO-ONDES

(30) Priority: 13.03.2020 US 202062989297 P
(43) Date of publication of application: 18.01.2023
(73) Proprietor: Biocompatibles UK Limited, Camberley, Surrey GU15 3YL (GB)
(72) Inventor: DAVIS, Ian, Stillwater, Minnesota 55082 (US); MUELLER, Kevin L., Woodbury, Minnesota 55129 (US); CHEN, Ji, Woodbury, Minnesota 55125 (US); GRANATA, Alysa, Louis Park, Minnesota 55416 (US); CONDIE, Catherine, Shoreview, Minnesota 55126 (US); KOLLMANN, Daniel, Andover, Minnesota 55304 (US); MOSESOV, Oleg, Maple Grove, Minnesota 55311 (US); BROWN, Andrew, Minneapolis, Minnesota 55414 (US)
(74) Representative: Vossius & Partner Patentanwälte Rechtsanwälte mbB
(86) International application number: PCT/US2021/022108
(87) International publication number: WO 2021/183888

(56) References cited:
- US-A- 5 843 144
- US-A1- 2002 000 234
- US-A1- 2003 171 745
- US-A1- 2010 049 031
- US-A1- 2010 241 115
- US-A1- 2014 275 993
- US-A1- 2020 046 414

## Description

### FIELD OF INVENTION

The present invention generally relates to tissue ablation devices

### BACKGROUND OF THE INVENTION

In the treatment of diseases such as cancer, certain types of tissues have been found to denature at elevated temperatures. These types of treatments, known generally as hyperthermia therapies, typically utilize electromagnetic radiation to heat cancerous tissue to temperatures above 60°C while maintaining healthy tissue at lower temperatures where irreversible cell destruction will not occur. Microwave ablation is one of such treatments utilizing electromagnetic radiation to heat tissue.

Microwave tissue ablation is a less invasive procedure than surgical removal and may be in many situations when tumors are difficult to remove by surgery, for example when the tumor is relatively small, disposed close to a relatively small organ, or disposed close to a major blood vessel. The approach has been used in organs such as the prostate, heart, and liver, where surgical removal of tumors may be difficult to perform. In this context, documents US 5 843 144 A and US 2020/046414 A1 are referred to.

In order to effectively plan and optimize the procedure, it is desired that the ablation device causes predictably sized and shaped volumes of ablation. For this reason, regularly shaped, predictable ablation volumes are preferred, and it is particularly preferred to produce spherical, or near spherical ablation volumes. An ablation device with predictably sized and shaped ablation volumes simplifies the surgical procedures and reduces the undesirable medical complications.

One of the potential undesirable complications related to microwave tissue ablation is the creation of steam pockets, which can be formed when water found within the cells of the tissue being heated by the microwave ablation is vaporized and turns to steam. If enough steam builds within the tissue, the steam pockets may burst. The bursting of steam pockets, which is commonly referred to as steam pops, causes discomfort to patients and results in other complications.

### SUMMARY

The invention is defined in the appended set of claims. Methods mentioned hereinafter do not form part of the present invention. The ablation system includes an ablation device configured to provide energy to a target area; an ablation generator configured to provide power to the ablation device; and a controller in communication with the ablation generator and being configured to cause the ablation generator to apply a first power level to the ablation device at a first time and cause the ablation generator to apply a second power level to the ablation device at a second time, the second power level being higher than the first power level and the second time being after the first time; the power provided by the ablation generator to the ablation device is adjusted from the first power level to the second power level between the first time and the second time, adjusting the power from the first power level to the second power level includes causing the ablation generator to provide a plurality of distinct intermediate power levels corresponding to delivering a plurality of intermediate energy levels from the ablation device to the target area, and each of the intermediate power levels is between the first power level and the second power level, and each consecutive intermediate power level is greater than the previous intermediate power level.

The ablation generator is configured to provide power at each of the intermediate power levels for a predetermined amount of time.

The plurality of distinct intermediate power levels are incremented using a step up function.

Preferably, the first power level is between 20W and 60W and the second power level is between 70W and 150W.

The time between the first time and the second time is between 40 seconds and 100 seconds.

Each intermediate power level is between 1W and 10W greater than the previous intermediate power level.

Preferably, the system further includes a coolant system for the ablation device, the coolant system includes a coolant chamber, the coolant chamber surrounding a distal portion of the ablation device; a coolant inlet, the coolant inlet configured to provide a coolant to the coolant chamber; a coolant outlet, the coolant outlet configured to carry the coolant away from the coolant chamber; and one or more pumps configured to supply the coolant to the coolant chamber.

Preferably, the system further includes a display configured to provide information regarding an ablation procedure; a user interface; and the controller is in communication with the display and the user interface, and is further configured to receive inputs from the user interface, the received inputs include at least one of: a total ablation time, a total ablation energy, and a dimension for an ablation zone, determine ablation procedure parameters based on the received inputs, and cause the ablation generator to apply ablation power to the ablation device based on the ablation procedure parameters.

Preferably, the user interface is further configured to receive inputs regarding the ablation procedure parameters during the ablation procedure.

Preferably, the received inputs during the ablation procedure include at least one of: an input to increase the total ablation time, and an input to terminate the ablation procedure.

Preferably, the received inputs during the ablation procedure include an input to adjust the power level of the ablation power.

Preferably, the display is further configured to display at least one of: a duration of the ablation procedure, a percent of total energy delivered, a current power level of the ablation power, and a power level of the ablation energy over time.

Preferably, the system further includes a second ablation device configured to provide energy to the target area; the ablation generator configured to provide power to the second ablation device; and the controller further configured to cause the ablation generator to apply a third power level to the second ablation device at a third time, the third power level corresponding to delivering a third level of energy from the second ablation device to the target area, and cause the ablation generator to apply a fourth power level to the ablation device at a fourth time, the fourth power level corresponding to delivering a fourth level of energy from the second ablation device to the target area, the fourth power level being higher than the third power level and the fourth time being after the third time; the power provided by the ablation generator to the second ablation device is adjusted from the third power level to the fourth power level between the third time and the fourth time, adjusting the power from the third power level to the fourth power level includes causing the ablation generator to provide a plurality of distinct second ablation device intermediate power levels corresponding to delivering a plurality of second ablation device intermediate energy levels from the second ablation device to the target area, each of the second ablation device intermediate power levels is between the third power level and the fourth power level, and each consecutive second ablation device intermediate power level is greater than the previous second ablation device intermediate power level; and the ablation generator provides power at each of the intermediate power levels and second ablation device intermediate power levels for a predetermined amount of time.

Preferably, the first power level is the same as the third power level and the second power level is the same as the fourth power level.

Preferably, the first time and third time are approximately the same; and the second time and fourth time are approximately the same.

Preferably, a tissue ablation system includes an ablation device configured to provide energy to a target area; an ablation generator configured to provide power to the ablation device, and a controller in communication with the ablation generator and being configured to cause the ablation generator to apply a first power level to the ablation device at a first time and cause the ablation generator to apply a second power level to the ablation device at a second time, the second power level being higher than the first power level and the second time being after the first time; the power provided by the ablation generator to the ablation device is adjusted from the first power level to the second power level between the first time and the second time, adjusting the power from the first power level to the second power level includes causing the ablation generator to provide a plurality of distinct intermediate power levels corresponding to delivering a plurality of intermediate energy levels from the ablation device to the target area, each of the intermediate power levels is between the first power level and the second power level, each consecutive intermediate power level is greater than the previous intermediate power level, and the ablation generator provides power at each of the intermediate power levels for a predetermined amount of time,

Preferably, the plurality of distinct intennediate power levels are incremented using a step up function.

Preferably, the first power level is between 20W and 60W and the second power level is between 70W and 150W.

The time between the first time and the second time is between 40 seconds and 100 seconds.

Each intermediate power level is between 1W and 10W greater than the previous intermediate power level.

Preferably, the system further includes a coolant system for the ablation device, the coolant system including a coolant chamber, the coolant chamber surrounding a distal portion of the ablation device; a coolant inlet, the coolant inlet configured to provide a coolant to the coolant chamber; a coolant outlet, the coolant outlet configured to carry the coolant away from the coolant chamber; and one or more pumps configured to supply the coolant to the coolant chamber.

Preferably, the system includes a display configured to provide information regarding an ablation procedure; a user interface; the controller is in communication with the display and the user interface, and is further configured to receive inputs from the user interface, the received inputs include at least one of: a total ablation time, a total ablation energy, and a dimension for an ablation zone; determine ablation procedure parameters based on the received inputs; and cause the ablation generator to apply ablation power to the ablation device based on the ablation procedure parameters.

Preferably, the user interface is further configured to receive inputs regarding the ablation procedure parameters during the ablation procedure.

Preferably, the received inputs during the ablation procedure further include at least one of: an input to increase the total ablation time, and an input to terminate the ablation procedure.

Preferably, the received inputs during the ablation procedure further include an input to adjust the power level of the ablation power.

Preferably, the display is further configured to display at least one of: a duration of the ablation procedure, a percent of total energy delivered, a current power level of the ablation power, and a power level of the ablation energy over time.

Preferably, the tissue ablation system includes a first ablation device configured to provide energy to a target area; a second ablation device configured to provide energy to the target area; an ablation generator configured to provide power to the first ablation device and the second ablation device; a controller in communication with the ablation generator and being configured to cause the ablation generator to apply a first power level to the first ablation device at a first time, cause the ablation generator to apply a second power level to the ablation device at a second time, the second power level being higher than the first power level and the second time being after the first time, cause the ablation generator to apply a third power level to the second ablation device at a third time, the third power level corresponding to delivering a third level of energy from the second ablation device to the target area, and cause the ablation generator to apply a fourth power level to the ablation device at a fourth time, the fourth power level corresponding to delivering a fourth level of energy from the second ablation device to the target area, the fourth power level being higher than the third power level and the fourth time being after the third time; the power provided by the ablation generator to the first ablation device is adjusted from the first power level to the second power level between the first time and the second time, adjusting the power from the first power level to the second power level includes causing the ablation generator to provide a plurality of distinct first ablation device Intermediate power levels corresponding to delivering a plurality of first ablation device intermediate energy levels from the first ablation device to the target area, each of the first ablation device intermediate power levels is between the first power level and the second power level, and each consecutive first ablation device intermediate power level is greater than the previous first ablation device intermediate power level; the power provided by the ablation generator to the second ablation device is adjusted from the third power level to the fourth power level between the third time and the fourth time, adjusting the power from the third power level to the fourth power level includes causing the ablation generator to provide a plurality of distinct second ablation device intermediate power levels corresponding to delivering a plurality of second ablation device intermediate energy levels from the second ablation device to the target area, each of the second ablation device intermediate power levels is between the third power level and the fourth power level, and each consecutive second ablation device intermediate power level is greater than the previous second ablation device intermediate power level, and the ablation generator provides power at each of the first ablation device intermediate power levels and second ablation device intermediate power levels for a predetermined amount of time,

Preferably, the first power level is the same as the third power level and the second power level is the same as the fourth power level.

Preferably, the first time and third time are approximately the same; and the second time and fourth time are approximately the same.

Preferably, the system further includes a display configured to provide information regarding an ablation procedure; a user interface; and the controller is in communication with the display and the user interface, and is further configured to receive inputs from the user interface, the received inputs including at least one of: a total ablation time, a total ablation energy, and a dimension for an ablation zone, determine ablation procedure parameters based on the received inputs, and cause the ablation generator to apply ablation power to the first or second ablation device based on the ablation procedure parameters.

The system may be used in a method including the steps of providing ablation power at a first power level at a first time, the first power level corresponding to delivering a first level of energy from an ablation device to a target area; providing ablation power at a second power level at a second time, the second power level corresponding to delivering a second level of energy from the ablation device to the target area, the second power level being higher than the first power level and the second time being after the first time; and providing a plurality of distinct intermediate ablation power levels to the ablation device between the first time and the second time corresponding to delivering a plurality of distinct intermediate energy levels from the ablation device to the target area, each of the intermediate power levels is between the first level of ablation power and the second level of ablation power, and each consecutive intermediate power level is greater than the previous intermediate power level,

Preferably, the method includes receiving inputs from a user interface, the received inputs including at least one of: a total ablation time, a total ablation energy and a dimension for an ablation zone; determining ablation procedure parameters based on the received inputs, the ablation procedure parameters including determining a third time, the third time being after the second time; and providing the second power level to the ablation device between the second time and the third time,

Preferably, the method further includes receiving inputs between the first time and the third time, the received inputs between the first time and the third time including at least one of: an input to increase the total ablation time, and an input to terminate an ablation procedure.

Preferably, the method further includes receiving inputs between the first time and the third time, the received inputs between the first time and the third time including an input to adjust an applied level of the ablation power,

Preferably, the method further includes displaying a delivered energy during the ablation procedure on a display.

While multiple embodiments are disclosed, still other embodiments of the presently disclosed subject matter will become apparent to those skilled in the art from the following detailed description, which shows and describes illustrative embodiments of the disclosed subject matter. Accordingly, the drawings and detailed description are to be regarded as illustrative in nature and not restrictive.

### BRIEF DESCRIPTION OF DRAWINGS

Advantages of the present invention may become apparent to those skilled in the art with the benefit of the following detailed description and upon referesice to the accompanying drawings.
FIG. 1A shows a block diagram including components of a system for performing an ablation process according to one embodiment of the disclosure,
FIG. 1B shows a block diagram demonstrating operation of an ablation device interface for interfacing with an ablation device for performing an ablation process according to one embodiment of the disclosure.
FIG. 2 is a simplified illustration of a cooling system according to the disclosure.
FIG. 3 is a perspective view of a microwave tissue ablation device with a handle according to one embodiment of the disclosure.
FIG. 4A is a perspective view of a microwave tissue ablation device 400 according to one embodiment of the disclosure.
FIG. 4B is a sectional view across the line X-Y to illustrate one embodiment of the cooling features.
FIG. 5 is a side view of a microwave tissue ablation device according to one embodiment of the disclosure.
FIG. 6A shows a plain view of a plurality of microwave ablation needle configurations.
FIG. 6B shows an elevation view of a plurality of ablation devices at different depth arrangements.
FIG. 7 shows an exemplarily ablation procedure using a Step-Up Mode.
FIG. 8 shows an example sequence of duty cycling ablation devices.

### DETAILED DESCRIPTION OF THE INVENTION

The size and dimension of an ablation area created by the microwave tissue ablation device is dependent, among other factors, on the type of microwave antenna. Clinicians may select a microwave antenna capable of generating an ablation region greater than the size and dimension of the target tissue and insert the microwave antenna such that the ablation region created by the microwave antenna includes the target tissue. Where the tissue to be ablated is larger than the size of the ablation volume produced by the device, more than one device may be used and the ablation volumes combined to cover the tissue to be ablated. The embodiments of the microwave tissue ablation device described herein may be used to create predictably shaped ablation regions, with reduced tailing which aids ablation planning and prevents damage to tissue outside the volume to be treated.

In some embodiments, ablation devices disclosed herein are microwave ablation devices configured to cause ablation by emission of microwave energy, which kills the tissue by heating. Typically the devices are microwave ablation needles having microwave antennas such as those described herein.

In a further aspect, the invention provides a system for microwave ablation of tissue comprising one or more microwave ablation devices such as probes or needles as described herein, the microwave ablation device comprising a microwave antenna configured to transmit microwave energy to tissue, a microwave generator configured to provide microwave energy to the microwave antenna via a feedline, one or more power cables configured to connect the microwave generator to the microwave antenna of the ablation devices and to deliver microwave energy provided by the microwave generator to the antenna for the ablation of tissue.

Ablation devices such as those described herein can be configured to operate at powers of up to 150 watts and for periods of up to 20 minutes or more. The devices heat up during use due to resistive heating of the antenna and to energy reflected from the tissue and therefore typically at least the distal portion of the device including a distal portion of the feedline and the antenna will require cooling. Conveniently, in various embodiments, the whole feedline and antenna are cooled. Cooling the antenna prevents the device itself from becoming damaged and prevents tissue close to the antenna becoming over heated or charred. This alters the physical properties of the tissue, including its energy absorption and reflection characteristics and therefore reduces the efficiency of the antenna and may alter the ablation zone. In an embodiment the tissue ablation devices above therefore may additionally comprise a cooling system to cool the antenna and/or at least a portion of the feedline. Such cooling systems are typically configured to pass a cooling fluid such as a coolant (e.g. water) over at least a portion of the feedline and over the antenna. Typically, such systems comprise a coolant inlet and a coolant outlet which cooperate to pass a coolant over the antenna and optionally at least a portion of the feedline to cool the antenna and optionally at least a portion, preferably all, of the feedline. The antenna and feedline are typically in contact with the coolant.

In one option the cooling system comprises a coolant chamber surrounding the antenna and at least a distal portion of the feedline and having a coolant inlet conduit, configured to supply coolant to the coolant chamber and a coolant outlet conduit configured to carry coolant away from the coolant chamber, the coolant inlet and coolant outlet conduits configured to pass coolant over at least a portion of the feedline and at least a portion of the antenna.

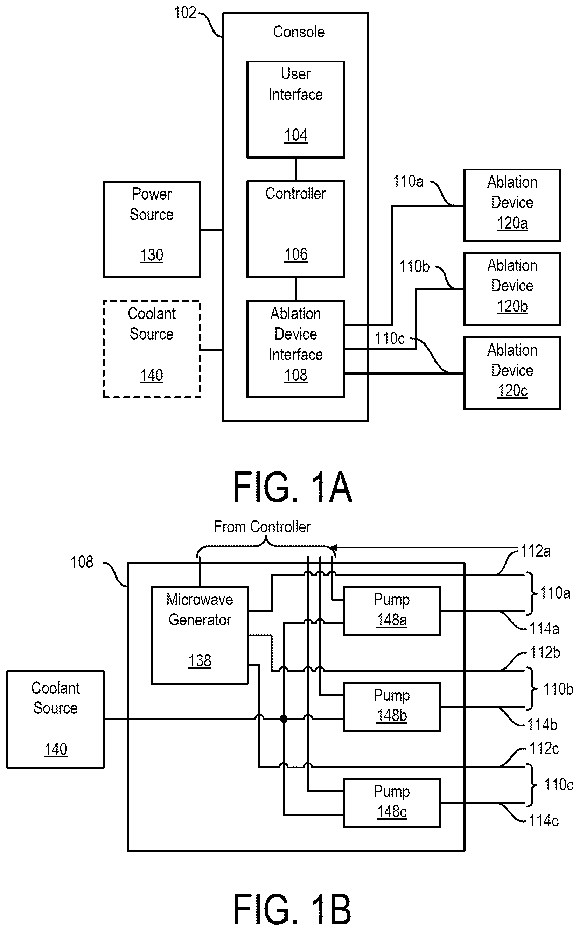

FIG. 1A shows a block diagram including components of a system for performing an ablation process according to one embodiment of the disclosure. The system includes a console 102 including a user interface 104, controller 106, and an ablation device interface 108. In an embodiment, user interface 104 includes a display for presenting information to a user and an input device for receiving inputs from the user, such as via one or more buttons, dials, switches, or other actuatable elements. In an embodiment, user interface 104 comprises a touchscreen display that functions as both the display and the input device of the user interface 104.

According to an aspect of the invention, the ablation device interface 108 of the console 102 is arranged to interface with one or more ablation devices. In the embodiment of FIG. 1A, ablation device interface 108 interfaces with three ablation devices 120a, 120b, 120c via lines 110a, 110b, 110c, respectively. In an embodiment, a console 102 can interface one, two, or all three ablation devices (120a, 120b, 120c) individually or simultaneously. It will be appreciated that, while three ablation devices are shown in the embodiment of FIG. 1A, different aspects of the invention may include a console having an ablation device interface capable of interfacing with different numbers of ablation devices.

In an embodiment, a console includes an ablation device interface capable of interfacing with a single ablation device. In other embodiments, a console includes an ablation device interface capable of interfacing with two ablation devices, with three ablation devices, with four ablation devices, or with five ablation devices. In some examples, an ablation device interface can be configured to interface with any number of ablation devices.

According to certain aspects of the invention, a console can be used to operate any number of ablation devices up to the number of ablation devices supported by the ablation device interface. For example, a console having an ablation device interface capable of receiving three ablation devices simultaneously can be configured to operate one, two, or three ablation devices.

In an embodiment, lines 110a, 110b, 110c are configured to provide a coolant (e,g., from a coolant source 140) and ablation power (e.g., microwave signals) to ablation devices 120a, 120b, 120c, respectively. Lines 110a, 110b, 110c can be configured to provide a path for a coolant to be provided to a respective ablation device and a return path for receiving coolant from the respective ablation device after having traversed a coolant flow path within the ablation device.

According to an aspect of the invention, the controller 106 is configured to interface with the user interface 1 04 and the ablation device interface 108. In an embodiment, the controller 106 can be configured to receive one or more inputs via the user interface 104 and output one or more items via the user interface 104.

The controller 106 can be configured to control operation of one or more ablation devices (e.g, 120a, 120b, 120c) via the ablation device interface 108. In an embodiment, controller 106 can cause coolant to be provided to one or more ablation devices via the ablation device interface 108. The controller 106 can cause ablation power to be provided to one or more ablation devices in order to cause the ablation device to perform an ablation process. In an embodiment, the ablation power provided to an ablation device causes a microwave ablation device to emit microwave radiation. A power source 130 can provide electrical power used to generate the ablation power.

In an example, the controller includes one or more processors and memory comprising instructions for causing the one or more processors to be performed via the controller. In various embodiments of the invention, a controller may be implemented as one or more processors, such as one or more microprocessors, digital signal processors (DSPs), application specific integrated circuits (ASICs), field programmable gate arrays (FPGAs), programmable logic circuitry, or the like, either alone or in any suitable combination. A controller may also include memory that stores program instructions and related data that, when executed cause the controller to perform the functions attributed thereto in this disclosure. Memory may include any fixed or removable magnetic, optical, or electrical media, such as RAM, ROM, CD-ROM, flash memory, EEPROM, or the like. Memory may also include a removable memory portion that may be used to provide memory updates or increases in memory capacities. A removable memory may also allow image data to be easily transferred to another computing device. A controller may also be implemented as a System on Chip that integrates some or all components of a computer or other electronic system into a single chip.

FIG. 1B shows a block diagram demonstrating operation of an ablation device interface for interfacing with an ablation device for performing an ablation process according to one embodiment of the disclosure. In an example, an ablation device interface 108 includes one or more fluid pumps, each of the one or more fluid pumps (148a, 148b, 148c) being configured to pump a coolant to a respective ablation device. For example, as shown, pump 148a is in communication with coolant source 140, and can be configured to provide coolant to an ablation device (e.g, 120a) via a coolant line 114a. Such pump(s) can be controlled by the controller. The controller can be configured to control the flow rate of fluid provided from a pump (e.g., 148a) to an ablation device (e.g., 120a), including initiating the pump providing the coolant to the ablation device and stopping the pump providing the coolant to the ablation device.

In the example of FIG. 1B, the ablation device interface 108 includes three pumps 148a, 148b, 148c for providing coolant to a respective ablation device via coolant lines 114a, 114b, 114c, respectively. Coolant lines 114a, 114b, 114c can be included in lines 110a, 110b, 110c shown in FIG. 1A, respectively. In an embodiment, each pump is controlled by the controller and independently from the other pumps, for example, whereby any pump can operate independently of the operating status of the other pumps.

In another embodiment, each of pumps 148a, 148b, 148c comprises a peristaltic pump driven by a single motor controlled by the controller. In some such examples, each pump operates at the same rate defined by the motor, and coolant flows through any connected ablation devices via coolant lines 114a, 114b, 114c. The controller can adjust the flow rate of coolant through the ablation devices by controlling the speed of the motor.

In some examples, coolant provided to the ablation device is provided in a closed loop recirculation system, wherein coolant is received from the ablation device and returned to the coolant source 140. In an embodiment, coolant source 140 comprises a reservoir of coolant, such as sterile water, from which coolant is drawn, directed to one or more ablation devices via a coolant line, and returned to the reservoir from the one or more ablation devices via a coolant outlet line configured to carry coolant away from the ablation device. In some alternate examples, coolant outlet line(s) carry coolant away from the ablation device toward a waste system (e.g., toward a drain).

The ablation device interface of FIG. 1B includes a microwave generator 138 for generating and providing microwave signals to a microwave antenna in a microwave ablation device configured to transmit microwave energy to tissue. Providing microwave signals to the ablation device can include providing ablation power to the ablation device such that the device emits microwave radiation. Microwave generator 138 can provide microwave signals to ablation devices via power cable. In the embodiment of FlG. 1B, microwave generator 138 can provide microwave signals to up to three ablation devices via power cables 112a, 112b, 112c, respectively.

Power cables 112a, 112b, 112c are preferably coaxial cables which are preferably rated to at least 30 watts, preferably at least 100 watts, preferably at least 150 watts power. The cables may be cooled cables configured to be cooled by a coolant supply, preferably by circulating coolant along the cable between a cable coolant inlet and a cable coolant outlet. In some examples, coolant lines 114a-c provide coolant along power cables 112a-c, respectively. In an example configuration, the system comprises a cooling system and the cooling system is configured to cool both the cable and the microwave ablation device.

In some examples, the microwave generator is preferably configured to supply microwave energy to the antenna in one or more of the 915MHz band (902 to 928 GHz), the 2.45 GHz band (2.402 to 2.483 GHz), or the 5.8GHz band (5.725 to 5.875 GHz), preferably in the 2.45 GHz band and most preferably at or about 2.45 GHz. The microwave generator may be configured to provide microwave energy to the antennas of up to 5 microwave ablation probes, preferably of one, two or three probes.

The microwave generator 138 can be configured to provide microwave signals prescribed by the controller 106. For example, in an example embodiment, the controller 106 can instruct the microwave generator 138 to provide particular microwave signals to a particular ablation device. The controller can be configured to designate a particular ablation magnitude (e.g., desired microwave power and/or energy emitted from ablation device, etc.), ablation duration, or other parameters, such as a duty cycle, phase shift, or other parameters associated with the microwave signal. In some examples, the microwave signal includes an electrical power (e.g., 90 W) delivered to the ablation device. The microwave signal can include an electrical signal including properties (e.g., electrical power, frequency, etc.) in order to cause the ablation device to emit microwave radiation having desired characteristics (e.g, microwave power radiated to surrounding tissue, etc.). The electrical signal can provide a desired ablation power to the microwave ablation device.

**In** an embodiment, the controller 106 can instruct the microwave generator 138 to apply microwave signals to each of a plurality of ablation devices. For example, with respect to FIG. 1B, the controller can instruct the microwave generator 138 to provide a first microwave signal to a first ablation device via power cable 112a, provide a second microwave signal to a second ablation device via power cable 112b, and provide a third microwave signal to a third ablation device via power cable 112c. In some such examples, the microwave generator 138 can provide such first, second, and third microwave signals simultaneously. Such signals can be the same signal or different signals. For example, in an embodiment, the same level of ablation power is provided by each of the first, second, and third microwave signals.

In some examples, the controller may be configured to control one or more of the following parameters: the output wavelength, the output power, the time period over which microwave energy is delivered to one or more of the antennas, the time period over which energy is delivered at an output power. Where the ablation device comprises a sensor, such as a temperature sensor, the controller can be configured to control any one or more of the parameters in response to a signal from the sensor (e.g., a temperature measurement). For example the controller may be configured to switch off the power to one or more of the antennas in response to an over temperature condition.

While shown in FIG. 1B as being implemented as a single microwave generator 138 configured to provide microwave signals to a plurality of ablation devices, in some examples, an ablation device interface 108 can include a plurality of microwave generators, each corresponding to a respective ablation device. In an embodiment, the controller 106 is in communication with a plurality of microwave generators and can be configured to cause the plurality of microwave generators to apply microwave signals to respective power cables (e.g., 112a, 112b, 112c) to provide such microwave signals to respective ablation devices.

FIG. 1B shows an example embodiment wherein three lines 110a, 110b, 110c can provide microwave signals and coolant to a respective three ablation devices simultaneously. In some aspects of the invention, microwave signals and coolant can be provided to a subset of lines 110a, 110b, 110c, for example, if fewer than three ablation devices are connected to the console 102. Further, in some aspects, microwave signals and coolant can be provided to a subset of lines 110a, 110b, 110c even if three ablation devices are connected to the console 102. For example, one or more such connected ablation devices can remain unused.

In an embodiment, controller 106 controls which ablation devices (e.g, which lines of 110a, 110b, 110c) receive microwave signals and coolant. In an aspect of the invention, the controller 106 can control aspects of the microwave signal, such as magnitude, frequency, duty cycle, duration, etc. of the microwave signal. In another aspect of the invention, the controller 106 can control aspects of providing the coolant to an ablation device, such as controlling a flow rate of the coolant, for example, by controlling operation of a respective pump. In an embodiment, for each ablation device, the controller controls aspects of both the microwave signal applied to an ablation device and aspects of providing the coolant to the ablation device. During operation, different ablation devices can each receive microwave signals and amounts of coolant independent of the signals and fluid received at other ablation devices, and can be the same as or different from microwave signals and amounts of fluid provided to other ablation devices.

While FIG. 1B shows an ablation device interface for interfacing with three ablation devices, it will be appreciated that a console according to different embodiments can include an ablation device interface capable of interfacing with a different number of ablation devices.

It will be appreciated that, while the block diagram of FIG. 1B shows an ablation device interface 108 including several components for interfacing with ablation devices, the components shown as being a part of the ablation device interface 108 are not necessarily contained within a single module or housing. Such components are grouped into the ablation device interface in that such components facilitate control of connected ablation devices by controller 106.

Additionally, while FIG. 1B shows an ablation device interface for interfacing with microwave ablation devices, it will be appreciated that similar ablation device interface concepts can be used to provide an interface between a controller and other ablation devices, such as RF ablation, cryoablation, or the like.

In an embodiment, the ablation device interface includes one or more ports configured to receive a portion of an ablation device, such as a cartridge having a fluid interface for connecting to a fluid line (e.g., 114a) and an electrical interface for connecting to a power cable (e.g., 112a).

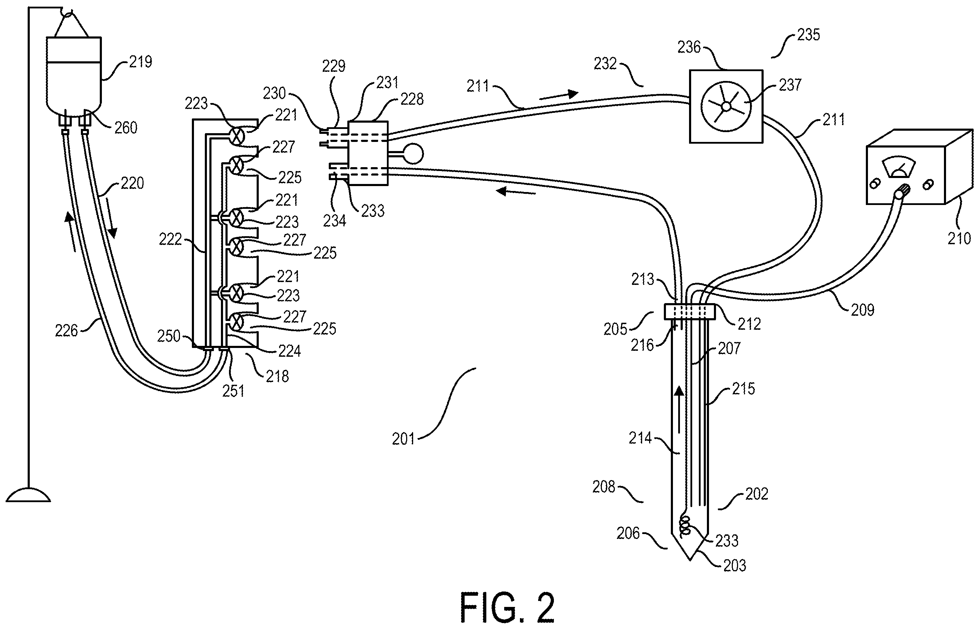

FIG. 2 is a simplified illustration of a cooling system according to the disclosure. The system 201 comprises an ablation device 202. In this case the microwave ablation device comprises a microwave ablation needle which is configured to deliver microwave energy to a patient's tissue to ablate the tissue.

The microwave ablation device 202 may have a tip 203 configured to penetrate tissue and an elongated shaft having a proximal end 205 and a distal end 206. The shaft encloses a coolant chamber 214 and a feedline 207, which may be a coaxial cable having an inner conductor, an outer conductor, and a dielectric therebetween (not shown in FIG. 2). The feedline of FIG. 2 comprises, distally, a radiating region 208 comprising a microwave antenna 204. The proximal end of the feedline 207 may be attached to a cable 209 (typically a coaxial cable) connecting the microwave ablation device 202 to a microwave generator 210 for providing microwave energy to the device. The cable may be releasably connectable, or, as in this case, permanently attached to the device. In some embodiments, as shown with respect to FIGS. 1A or 1B, the microwave generator 210 may be housed within a console, such as console 102.

The device is provided with coolant via a device coolant supply line 211 which may be permanently attached to the device coolant inlet 212. In some embodiments, the device coolant supply line may, alternatively, be releasably connectable to the coolant inlet 212 such as via a Luer^{®} type connector. The device coolant inlet 212 is in fluid communication with the device coolant outlet 213, via a series of coolant passageways 214, 215, and 216 configured to circulate coolant within the device. In this simplified representation, coolant enters the device through the coolant inlet tube 215, circulates through a coolant chamber 214 to cool the device, and leaves via the coolant outlet tube 216 and device coolant return line 217.

System 201 is provided with a manifold 218 which receives coolant fluid from a coolant fluid source 219, via a coolant system supply line 220. The coolant system supply line 220 may be permanently connected to the manifold 218 at the manifold fluid supply inlet 250 or it may be releasably connectable to the supply inlet 250, for example by a LaerLok^{®} connector. The coolant fluid source may be, for example, an IV bag. The in-flowing coolant may be distributed to one or more manifold outlet ports 21, via a manifold inflow conduit 222. In an advantageous embodiment, and is illustrated in FIG. 2, flow of coolant out of the port 221 may be controlled by a manifold outlet valve 223. This valve may be normally in the closed position. In some embodiments, as shown with respect to FIGS. 1A or 1B, the manifold 218 may be housed within a console, such as console 102.

The manifold 218 also comprises a manifold coolant outflow conduit 224 which provides a fluid connection between one or more manifold fluid inlet ports 225 and the coolant system return line 226. The coolant system return line 226 may be permanently connected to the manifold 218 at the manifold fluid return inlet 251 or it may be releasably connectable to the supply inlet 250, for example by a LuerLok^{®} connector. In an aspect of the design, a manifold inlet valve 227 controls the flow through each inlet port and may also normally be in the closed state.

A supply coupling 229 is configured for connection to a manifold outlet port 221. The system may also comprise a return coupling 233 which is configured for connection to a manifold inlet port. In one aspect, the manifold outlet valve 223 may be configured to open upon connection of the supply coupling 229. In one approach, the supply coupling may comprise projections 230 which cause the valve to open upon connection of the coupling 229, to the port 221, but other arrangements are possible as discussed elsewhere herein.

A coolant circuit coolant inlet 231 on the supply coupling 229 is in fluid communication with the device coolant supply line 211 so that connection of the supply coupling 229 to the outlet port 221 places the cooling circuit 232 in fluid communication with the cooling fluid source 219.

A return coupling 233 may have a coolant circuit outlet 234 in fluid communication with the device coolant return line 217. The supply coupling 229 and the return coupling 233 can be arranged for simultaneous connection to the manifold outlet port 221 and inlet port 225 respectively.

A pumping portion 235 may be arranged in the device cooling circuit 232 and may be arranged in the supply line 211 for example, and is arranged to circulate the coolant through the microwave ablation device 202. In the system shown in FIG. 2, the pump is a disposable pump head 236 having pump vanes 237, permanently connected in the device coolant supply line 211 and adapted to be connected to a pump head drive (not shown). Alternative pumping portions may be used and are described elsewhere herein. In some embodiments, as shown with respect to FIGS. 1A or 1B, the pumping portion 235 may be housed within a console, such as console 102.

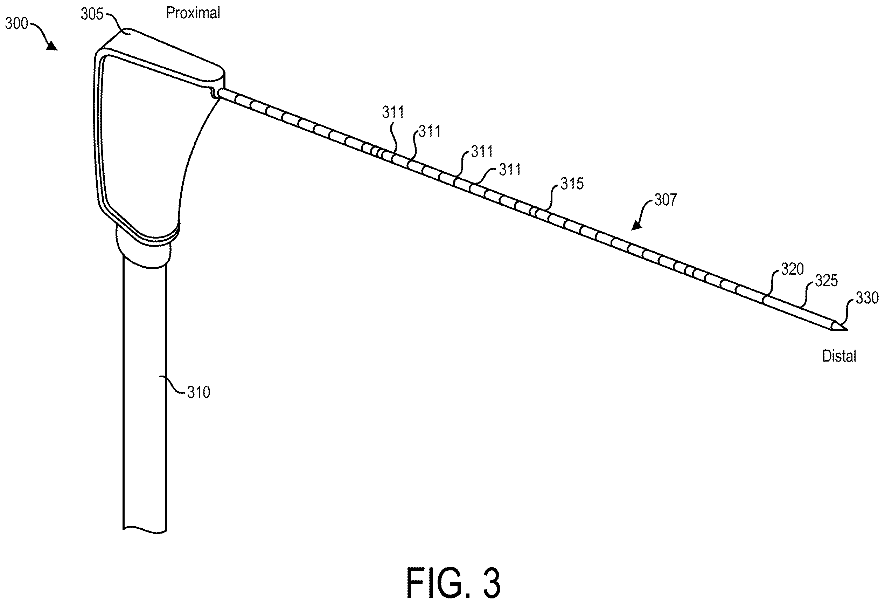

FIG. 3 is a perspective view of a microwave tissue ablation device 300 with a handle 305 according to one embodiment of the disclosure.

The microwave tissue ablation device 300 includes a handle 305. The handle 305 is configured to provide a firmer grip for a surgeon to handle the tissue ablation device 300. The handle 305 is further configured to house liquid manifolds for coolant circulation and coaxial connectors for powering the feedline.

The microwave tissue ablation device 300 includes a probe 307. The probe 307 is configured to be inserted into a patient's body for heating target tissue. In one embodiment, the probe 307 includes various ablation device components described elsewhere herein, such as the feedline, asymmetric dipole antenna, cooling system having inflow tubes and outflow tubes, etc. In an embodiment, the microwave antenna is configured to emit microwave radiation in a frequency band selected from the 915 MHz band (902 to 928 MHz), the 2.45 GHz band (2.402 to 2.483 GHz), and/or the 5.8 GHz band (5.725 to 5.875 GHz). The preferred wavelength is within the 2.45 GHz band and particularly the antenna is preferred to be configured to emit microwave energy at or about 2.45 GHz. The devices are configured to operate at up to 150 watts power supplied to the antenna,

The probe 307 includes a surface 315. The surface 315 is configured to be in contact with human tissue and is made with biocompatible materials. The device shaft is at least partially, metal, e.g., stainless steel, and includes markings 311, e.g., laser markings. The markings 311 are configured to inform the surgeon of the depth of the probe penetration into the body. It may comprise a lubricious surface layer such as PTFE, to aid insertion and prevent tissue sticking to the needle shaft while the needle is being inserted or extracted.

The shaft is typically cylindrical and is typically made of a biocompatible polymer, a biocompatible composite material, such as glass fiber reinforced polymer or carbon fiber reinforced polymer, ceramic or metal (such as stainless steel). The shaft is preferably made of ceramic or metal, but in a preferred embodiment the shaft comprises a metallic portion and a non-metallic portion. The non-metallic portion may be a biocompatible composite material, such as glass fiber reinforced polymer or carbon fiber reinforced polymer or ceramic, but is preferably ceramic due to its improved performance and strength. The ceramic is preferably an alumina or zirconia ceramic.

The shaft preferably terminates distally in a device cap. The shaft is preferably cylindrical. The feedline and antenna are preferably disposed within the device shaft. The device shaft typically extends from a proximal hub and terminates distally in a distal cap. The hub comprises electrical connections to electrical components of the shaft such as the feedline, and may also comprise coolant inlet and outlet connections, where necessary.

The diameter of the shaft is not limited, and is typically adapted for the intended purpose, for example for ablation needles, it is important to have a narrow needle to limit damage caused at insertion and to provide fine control of positioning, consequently the needle shaft is between 1.4 and 3 mm in diameter, preferably between 1.5 and 2.5 mm, particularly 2 to 2.5 mm.

The probe 307 of FIG. 3 includes an applicator cap 330. In an embodiment, applicator cap 330 is made of a biocompatible metal or a ceramic, e.g., preferably stainless steel or a ceramic. The applicator cap 330 can include a circular base and a distal tip (e.g., a trocar tip). The applicator cap 330 tip can include a sharp end disposed at a distal end of the applicator cap 330 and configured for penetration of tissue. The circular base can be configured to be sealed with a sheath of the probe 307 such that the interior of the probe 307 is fluidly isolated from the exterior of the probe 307.

The shaft may further comprise an echogenic region on the outer surface configured to be visible under ultrasound imaging. In one embodiment, this region comprises a coating comprising acoustically-reflective microspheres. The echogenic region extends at least to cover the region of the shaft radially outward of the antenna. The probe 307 of FIG. 3 includes an echogenic region 325 configured to be visible under ultrasound imaging and one embodiment, comprises a coating comprising acoustically-reflective microspheres.

Where the shaft comprises a metallic portion and a non-metallic portion, the joint between the two portions, where the metallic portion and the non-metallic portion abut, may be a point of potential weakness, especially where the non-metallic portion is ceramic, since ceramic is typically less flexible and more brittle than metals such as stainless steel. It is therefore preferred the shaft additionally comprises a resilient element between this portion and the metallic portion configured to provide resilience to the joint between the non-metallic (e.g., ceramic) portion and the metallic portion of the probe shaft in use.

The probe 307 further includes a region 320 configured to relieve strain on the probe induced during use, such as that caused by flexing of the shaft. This strain relief region is particularly useful when the distal portion of the probe sheath is ceramic. The strain relief region 320 is configured to provide the probe 307 added flexibility avoiding fracture of the probe 307 during a medical operation.

Although a resilient element may also be present between a non-metallic region and the cap, it is not necessary since the strains on the shaft at this point are lower. The resilient element may, for example, comprise a resilient annular spacer, which may be made of a resilient thermoplastic elastomer, such as polyether block amide (PEBA) - tradename PEBAX^{®} or Vestimid ^{®}E (Evonik Industries) or a polyaryletherketone (PAEK) such as Polyetheretherketone (PEEK), The spacer is preferably shaped and configured to space apart the proximal end of the non-metallic portion from the distal end of the metallic portion. The resilient element preferably abuts the metallic portion on a proximal face and the non-metallic portion on a distal face. The resilient annular spacer typically extends radially outward to form a surface flush with the outer surface of the probe shaft. The radially inner portion of the annular spacer may be extended proximally and/or distally to provide an annular step configured to support the inner face of the the proximal end of the non-metallic portion and/or the distal end of the metallic portion. In one preferred embodiment, the annular spacer is extended proximally to provide an annular step configured to support the inner face of the distal end of the metallic portion, but does not extend distally. The device shaft may also comprise an adaptor sleeve to support the joint between the non-metallic portion and the metallic portion of the shaft. The adaptor may be configured to take account of any differences in thickness between the non-metallic portion and the metallic portion of the shaft, such as to provide a smooth surface transition between the metallic and non-metallic portions. It may be metallic, or non-metallic such as a thermoplastic elastomer, such as a PEBA PEBAX^{®} or Vestimid ^{®}E or a PAEK such as PEEK. The adaptor is particularly important where the non-metallic portion is ceramic due to the thickness required for additional strength of the ceramic and the danger of flexing of the shaft causing cracking at this point. Conveniently the sleeve extends each side of the joint sufficiently to provide support for the joint and is typically positioned radially inward of the shaft, typically between the feedline and the inner wall of the shaft The adapter sleeve is preferably metallic.

The resilient element and the adaptor sleeve together comprise a strain relief region. The resilient element and the adaptor sleeve may be a single piece or separate.

In one preferred embodiment, the strain relief region comprises a resilient element as described above, which comprises a resilient annular spacer shaped and configured to space apart the proximal end of the non-metallic portion from the distal end of the metallic portion, the spacer configured to abut the metallic portion on a proximal face and the non-metallic portion on a distal face, the spacer extending radially outward to form a surface flush with the outer surface of the probe shaft, the radially innermost portion of the spacer extending proximally to provide an annular step configured to support the inner face of the distal end of the metallic portion; the strain relief region additionally comprising an adaptor sleeve extending each side of the joint and radially inward of the annular spacer. Preferably the sleeve extends proximally of the annular spacer and is configured to be in contact with and support the inner face of the distal end of the metallic portion of the shaft; and preferably extends distally of the spacer and is configured to be in contact with and support the inner face of the proximal end of the ceramic portion of the shaft.

The microwave tissue ablation device 300 includes a housing 310. The housing 310 houses coaxial cables, fluid lines, electric lines, etc.

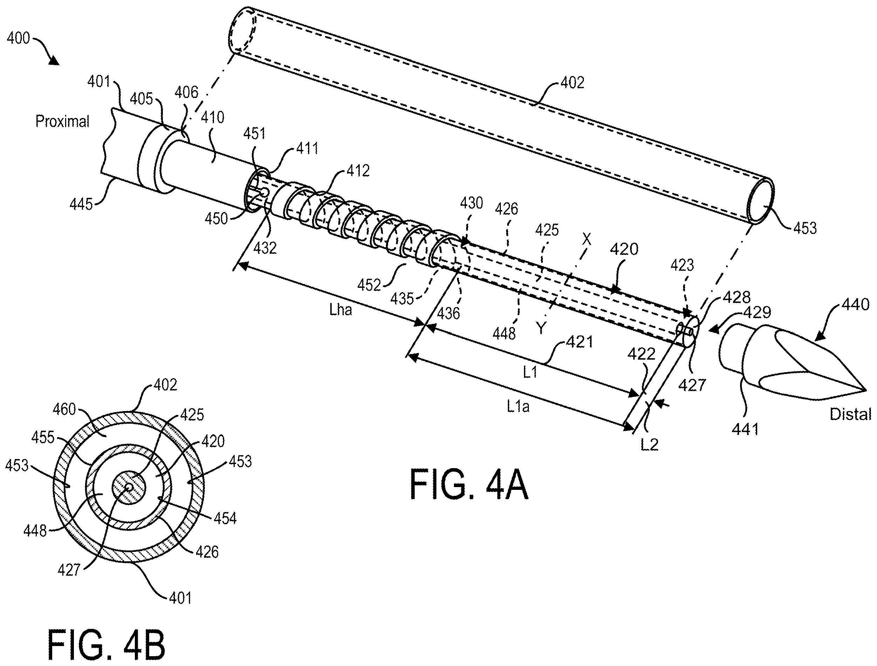

FIG. 4A is a perspective view of a microwave tissue ablation device 400 according to one embodiment of the disclosure. FIG. 4B is a sectional view across the line X-Y to illustrate one embodiment of the cooling features.

The tissue ablation device 400 of FIG. 4A has a shaft 401 having a metal portion 445 and a ceramic portion 402. The ceramic portion 402 extends from distal end 406 of a collar 405 to the base 441 of the cap 440. The ceramic portion 402 is shown separately from the shaft 401 in order to show the internal features of the device.

The tissue ablation device 400 includes a resilient element (e.g., collar 405), and an adaptor 410 to join the metal portion 445 to the ceramic portion 402 of the shaft. In devices of the invention, the adaptor takes up any difference in shaft thickness between the two portions and additionally acts to reduce flexing between the metal portion 445 and the ceramic portion 402. In devices of the invention, the resilient annular spacer between the ceramic portion and the metal portion of the shaft as shown here, acts to provide resilience to this region and to reduce the occurrence of fractures at this point due to strain on the shaft during use.

As described, for example, with respect to FIGS. 1A or 1B, microwave energy generated by a microwave generator can be supplied to the antenna by a power cable which electrically connects the microwave generator to the feedline 432 of the antenna 452 within the device 400. The microwave ablation devices also have a shaft surrounding and typically coaxial with both the microwave antenna and at least a distal portion of the feedline. The shaft typically extends from a proximal hub to a distal cap.

The feedline preferably comprises an inner conductor, an outer conductor, and a dielectric disposed therebetween. The feedline may comprise a further dielectric or insulator that insulates the outer conductor from other parts of the device and acts as an outer insulator to the feedline, but it is not required in all embodiments. In some embodiments the further dielectric may be absent from the distal portion of the feedline, at least up to the junction point. The feedline may lack such a further dielectric within the device shaft, such as between a proximal feedline connector of a distal hub, and the junction point of the antenna. The feedline is typically a coaxial cable having a central conductor, surrounded by a first dielectric, or insulator, the first dielectric being surrounded by the second conductor, which may be covered by the further dielectric or insulator as described above. The inner conductor is typically the power conductor.

In the example of FIG. 4A, tissue ablation device 400 has an antenna 452 including a helical arm 412, and a linear arm 420. A distal end 43 5 of the helical arm 412 forms an electrical connection with the outer conductor 430 of the feedline 432 at a junction point 436. In some embodiments, the junction point is conveniently towards, or at, the distal most end of the feedline. The feedline 432 may extend beyond the junction point in order to provide suitable mechanical support to the electrical junction, but preferably it not extend by more than 5 mm and particularly not more than 1 mm beyond the junction point.

Typically the helical arm is in the form of a single conductor. The helical arm of the antenna may be in the form of a wire or a ribbon, but is typically a wire having a circular cross section or a ribbon. The helical arm is preferably in the form of a cylindrical conductor, having a helical gap running from its proximal to its distal end to give a helical conductor having a planar conductor surface curved about the feedline. The helical arm does not make any other contact with either the inner conductor or the outer conductor, except at the junction point.

In the example of FIG. 4A, the helical arm 412 extends proximally from the junction point 436 in a series of turns about the feedline 432 and so is coaxially disposed about the feedline. The helical arm 412 forms no other electrical contact with the inner conductor 427 or the outer conductor 430, except the junction point 436. The helical arm may be affixed to its substrate by an adhesive in order to hold it in place and to make assembly easier. The helical arm may be embedded within a matrix such as a polymer layer or coating in order to protect it, to insulate it from the other parts of the device, or to provide a seal.

In some embodiments, the helical arm is not coiled in direct contact with the feedline. It may, for example form turns at a position radially displaced from the feedline. The helical arm is preferably coiled about a substrate that supports it. Where the feedline comprises an outer insulator, this outer insulator may be the substrate for the helical arm, which may form turns around the outer insulator. Alternatively the helical arm may, for example, be coiled about a tubular substrate, such as a cooling tube positioned about the feedline.

In some embodiments, the total number of turns (N) is in the range of 1-12 but is not limited to integers. In preferred embodiments, N is typically from 4 to 8. For each complete helical turn, the axial distance is a pitch (P), which can range from 0.7-1.5 mm, preferably, the pitch ranges from 1-1.5 mm and in a preferred embodiment, the pitch (P) of the helical arm is from 1.2-1.25 mm. The number of helical loops (N), pitch (P) can affect the output of microwave energy, the shape of the emission field and the energy absorption spectrum. The judicious selection of each variable in combination can afford an ablation device with advantageous properties for tissue ablation.

In the example of FIG. 4A, the helical arm is coiled on a tube 426, which extends from the hub (not shown), through the metal portion 445 of the shaft to the tip 428 of the antenna 452. The electrical connection between the helical arm 412 of the antenna and the outer conductor of the feedline 432 passes through the tube at the junction point 436. In the illustrated example, the helical arm 412 has a length (Lha). In some examples, the overall length of the helical arm (Lha) can range from 1 to 18 mm, preferably the helical arm ranges from 4 to 10 mm. In a preferred embodiment, the helical arm ranges from 4 to 7 mm.

The linear arm 420 is an extension of the inner conductor 427 of the feedline 432 and is surrounded by a dielectric layer 425, except for the second portion 423, which is free of dielectric.

The linear arm of the antennas described herein is a conductor that is electrically connected to the inner conductor of the feedline and extends distally therefrom preferably on an axis co-axial with the helical arm and/or the feedline. The conductor is preferably in the form of a straight wire. In a particularly preferred embodiment, the linear arm includes a first, proximal, insulated portion and a second distal non-insulated portion. Typically, the first portion is surrounded by a dielectric and a second portion, distal of the first portion is free of dielectric. The second portion extends to the tip of the arm. The dielectric surrounding the first portion of the linear arm, preferably extends from the distal end of the feedline. In its simplest form, the linear arm of the antenna may be an extension of the feedline's inner conductor. The dielectric may then be an extension of the dielectric disposed between the central and outer conductors of the coaxial feedline.

Preferably, the linear arm and the helical arm of the antenna are coaxial with the shaft of the ablation device, and thus the linear arm is coaxial with and extends distally from, the helical arm. As shown, the linear arm 420 of the asymmetric. dipole antenna of FIG. 4A has a length L1a. The linear arm includes a first portion L1 421 coated with an insulator, which is an extension of the first dielectric layer of the feedline 432 which is disposed between the inner conductor 427 and the outer conductor 430 and is not visible in this view. The linear arm 420 further includes a second portion 423 which has a length L2 422 and which is not coated with the insulator. In one embodiment, the second portion L2 422 is exposed to the circulating coolant.

In one aspect, the portion of the linear arm lacking dielectric is partially or completely inserted into the metal cap, but does not touch the cap. This can be achieved by creating an open pocket in the base of the cap into which this part of the antenna or a portion of it is inserted. The degree to which the exposed distal tip is inserted influences the shape of the distal portion of the energy field and hence the shape of the ablation zone.

Where the distance between the tip and cap is greater than 3 mm they are not considered to be sufficiently coupled to be useful in shaping the ablation, particularly at 2.45 GHz.

The linear arm 420 preferably has a length (Lla) of from 4 mm to 14 mm and preferably from 8 mm to 10 mm. The second, exposed portion 423 preferably has a length (L2) of from 0.1 mm to 2 mm, preferably from 0.3 mm to 0.5 mm.

Thus, in a preferred embodiment, the helical arm 412 of the antenna is in the form of a ribbon, having a length (Lha) of 1 to 18 mm and comprises 1 to 14 turns, the linear arm 420 of the antenna is 4 to 14 mm long and has a second, distal portion 423 lacking dielectric of 0.1 to 3 mm long, the portion lacking dielectric separated from the base of the cap by 0.2 to 3 mm.

In a more preferred embodiment, the helical 412 arm of the antenna is in the form of a ribbon, having a length (Lha) of 4 to 10 mm and comprises 4 to 8 turns, the linear arm 420 of the antenna is 7 to 10 mm long and has a second, distal portion 423 lacking dielectric of 0.3 to 0.5 mm long, the portion lacking dielectric separated from the base of the cap by 1 to 2 mm.

In a more preferred embodiment, the helical arm 412 of the antenna is in the form of a ribbon, having a length (Lha) of 4 to 6 mm and comprises between 3 to 5 turns. The linear arm 420 is 7 to 10 mm long having a second, distal portion 423 lacking dielectric 0.3 to 0.5 mm long, the portion lacking dielectric separated from the base of the cap by 1 to 2 mm, preferably by at or about 1.5 mm.

Where the shaft has a non-metallic portion (e.g., ceramic portion 402), the non-metallic portion preferably extends axially to cover the antenna and thus is at least co-extensive with the radiating portion of the antenna. In one embodiment, the non-metallic portion extends at least from the proximal most point of the helical arm to the distal end of the shaft. (e.g., the point of attachment of the tip of the device). The non-metallic portion extends axially and circumferentially such that the shaft is preferably non-metallic between the proximal and distal extent of the non-metallic portion.

A cap may be configured to seal the distal end of the device to prevent coolant leakage or tissue fluid penetration. The cap may be manufactured as a separate part and may be configured to be attached to the shaft. The cap is preferably configured to aid insertion into tissues and to penetrate the skin of a patient and so may, for example, come to a distal point, or be configured as a trocar. The cap 440 shown in FIG. 4A includes a trocar tip. The trocar tip of cap 440 can be made with stainless steel and/or ceramic.

In some examples, the cap may be made of any suitable biocompatible material such as a biocompatible polymer, composite, ceramic or metal such as stainless steel. Where the cap is metal, the cap and the distal end of the antenna (i.e. the distal end of the linear arm of the antenna) may be configured to be electromagnetically coupled, This can be done by adjusting the distance between the distal tip of the antenna and the cap so that they become electromagnetically coupled at the frequency and at the power at which the antenna is intended to operate. This effect can be used to tune the shape of the distal portion of the energy field generated by the antenna and hence the shape of the ablation zone. The cap and antenna need not, however be so coupled, i.e, the antenna may be electromagnetically decoupled from the cap. It is preferred that the tip and cap do not touch. In practice the gap between the tip and the cap is 0.2 mm or greater, particularly 0.2 mm to 3 mm and most preferably 1 to 2 mm. Most preferably it is at or about 1.5 mm.

The shape of the energy field and hence the ablation volume can also be influenced by the provision of a metallic sheath concentric with the feedline. The sheath is preferably cylindrical and extends over at least a portion of the feedline proximal to the antenna. The sheath may also extend over at least a portion of the antenna, but preferably it terminates at a point proximal to the distal most point of the helical arm of the antenna and does not extend over the antenna. Preferably the gap between the sheath and the distal most portion of the helical arm is at least 0.1 mm. The gap may be for example, between 0.1 to 2 mm or 0.1 to 1 mm, preferably it is at or about 0.5 mm. The sheath is preferably not placed on the outer surface of the shaft, but is preferably radially displaced from the feedline and coaxial with it. Preferably it is placed between the feedline and the inner wall of the shaft. In one arrangement, the metal sheath may be an adaptor sleeve as described elsewhere herein.

Preferably, a coolant chamber is defined between the inner walls of the device shaft The chamber may be bounded distally by the cap and maybe bound proximally by one or more proximal seals, which close the coolant chamber proximally. The one or more seals are preferably formed at the hub or at a point between the hub and the proximal portion of the helical arm of the antenna. The cooling system comprises at least one coolant inlet conduit configured to deliver coolant to the coolant chamber and at least one coolant outlet conduit to remove coolant from the chamber. The coolant inlet and coolant outlet conduits typically pass through the proximal seal. In one approach, the coolant inlet conduit is a coolant inlet tube configured to deliver coolant to a position adjacent to and radially outward of the antenna and or feedline. In this case, the coolant inlet tube is preferably disposed within the coolant chamber between the antenna and the inner wall of the shaft. Preferably it is displaced radially outward of the feedline.

In an alternative arrangement the cooling system comprises a coolant inlet conduit and a coolant outlet conduit, each conduit arranged about at least a portion of the feedline and a portion of the antenna. Each conduit arranged in the form of a helix, the coolant inlet conduit and the coolant outlet conduit being interdigitated one with the other to form a double helix. In one preferred arrangement, the cooling system comprises a pair of helical dividers arranged about the feedline and at least a part of the antenna in a double helix, each divider extending radially outward, towards the inner wall of the shaft and extending radially inward towards the antenna and/or the feedline such that the coolant inlet conduit and the coolant outlet conduit are formed between the two dividers and the coolant inlet conduit and coolant outlet conduit form a double helix. The dividers may be in the form of filaments or ribbons, or a combination of both. Where the dividers comprise a ribbon, the ribbon is preferably generally perpendicular to the inner shaft wall. The filaments may be formed of metal or of a resilient polymer. The dividers preferably extend to seal against the inner wall and at least a portion of the antenna and/or feedline.

The cooling system may additionally comprise a coolant mixing chamber in fluid communication with both the coolant inlet and coolant outlet conduits, such that the coolant inlet and coolant outlet are in fluid communication via the coolant mixing chamber. The coolant mixing chamber is preferably configured to allow coolant to pass over at least a portion of the antenna, particularly at least a portion of the linear arm of the antenna. The coolant mixing chamber is particularly configured to allow coolant to pass over the distal portion of the linear arm of the antenna and at least a portion of the cap.

Alternatively and preferably, the cooling system comprises a coolant chamber defined between the inner walls of the device shaft. The chamber may be bounded distally by the cap and proximally by a seal between the hub and the shaft, or at some point distal from the hub and between the antenna and the hub as previously described. The coolant chamber surrounds the antenna and at least a distal portion of the feedline.

In an embodiment, the cooling system further comprises a cooling tube disposed about the feedline, the cooling tube preferably extending distally about the feedline and preferably coaxial therewith. The cooling tube preferably divides the coolant chamber into a first cooling conduit 448 and a second cooling conduit 460, the first cooling conduit disposed between the feedline and the inner wall of the cooling tube and the second cooling conduit disposed between the outer wall of the cooling tube and the inner wall of the device shaft. The cooling tube preferably extends over the distal portion of the feedline and extends distally about at least a portion of the antenna, preferably the cooling tube extends at least to the tip of the linear arm of the antenna. A variety of materials are suitable for the cooling tube, but it is preferably non-metallic. Conveniently the cooling tube may be made of a thermoset polymer such as a polyimide or of a thermoplastic polymer resin such as polyethylene terephthalate (PET) or a fluropolymer such as polytetrafluroethylene (PTFE), or of a PAEK such as PEEK.

As described elsewhere herein, in the example of FIG. 4A, the helical arm is coiled on a tube 426. In an embodiment, the tube 426 defines a first cooling conduit 448 between the inner wall 454 of the tube 426 and the feedline 432 and a second cooling conduit 460 between the outer wall 455 of the tube 426 and the inner wall of the shaft 453. Coolant may be pumped through the space between the tube 426 and the feedline 432 to a mixing chamber 429 between the tube 426 and the cap 440 and returns in the space between the outside of the tube 426 and the ceramic portion 402 of the shaft, through the space 411 between the inside of the shaft and the adaptor 410 and back down the metal portion 445 of the shaft to the hub.

The helical arm of the antenna may be disposed within the first cooling conduit, for example, the distal portion of the feedline may comprise a second insulator as described above, and the helical arm of the antenna is wound directly about the feedline, the second insulator extending axially at least between the helical arm and the second conductor of the feedline. In this case, the cooling tube may extend to cover a portion of the helical arm, but preferably to cover the helical arm and a portion of the linear arm, but most preferably the cooling tube extends at least to the distal end of the antenna, such that the first cooling conduit extends at least to the tip of the antenna.

Otherwise the cooling tube extends to cover the distal portion of the feedline and a portion of the linear arm, but most preferably the cooling tube extends at least to the distal end of the antenna, such that the first cooling conduit extends at least to the tip of the antenna.

The cooling system may additionally comprise a coolant mixing chamber in fluid communication with both the first cooling conduit and the second cooling conduit, such that the first cooling conduit and the second coolant conduit are in fluid communication via the coolant mixing chamber. The coolant mixing chamber is preferably configured to allow coolant to contact a portion of the cap.

Either the first or the second cooling conduit may act as the coolant input conduit or coolant output conduit. The first and second cooling conduits are open at the distal end allowing the coolant to circulate through the coolant mixing chamber between the distal end of the cooling tube and the base of an applicator cap.

The cooling tube preferably extends proximally towards the hub. The first cooling conduit and second cooling conduits are in fluid communication with coolant input and output connectors of the hub, for the supply of coolant and discharge of coolant during use,

In a particularly preferred approach, the helical arm of the antenna, preferably in the form of a ribbon, is wound about the cooling tube. In this case, the helical arm is in electrical contact with the outer conductor of the feedline at the junction point and extends distally in a series of turns about the cooling tube as described above. In this case, the cooling tube preferably extends at least to the junction point of the antenna and feedline, preferably it extends to cover at least a portion of the linear arm, but most preferably the cooling tube extends to the tip of the linear arm, such that the first cooling conduit extends at least to the tip of the antenna. Preferably, the electrical contact between the distal end of the helical arm and the outer conductor of the feedline passes through the cooling tube

In this approach it is preferred that the outer insulator does not extend over the distal portion of the feedline. Preferably, it does not extend over at least the portion which extends from a point on the feedline immediately proximal of the helical arm of the antenna to the junction point. The outer insulator may be absent from the entire feedline within the shaft of the ablation device.

In embodiments in which the cooling system comprises a cooling tube as described above, the helical arm may be either a wire or a ribbon, but is most preferably a ribbon. The helical arm is preferably in the form of a cylindrical conductor, having a helical gap running from its proximal end to its distal end to give a helical conductor having a planar conductor surface disposed about the feedline and preferably coaxial with it.

The cooling systems described herein pass a coolant (e.g., water) over the feedline and at least a portion of the antenna, preferably the whole antenna. It is not necessary to insulate the antenna from the coolant for normal operation. In some embodiments described herein, parts of the feedline are lacking an outer insulator surrounding the feedline. The feedline may be lacking an insulator between the hub and the junction point or its whole length within the device shaft. The helical arm of the antenna may also lack any insulation, particularly where it is wound about a cooling tube.

The ablation devices described herein may additionally comprise one or more temperature sensors, such as a thermocouple, to measure the temperature at points along the shaft. Typically a thermocouple may be located within the cooling system and configured to measure the temperature of the coolant or of other parts of the device such as the feedline or device shaft during operation of the device. The tissue ablation device 400 of FIG. 4A may include a temperature sensor 450 housed next to the internal adaptor 410 and having an electrical connection 451 via the hub to the control unit.

As described elsewhere herein, ablation devices such as those described herein typically comprise a proximal hub as discussed briefly above. The hub typically comprises connectors for connecting the feedline to an energy supply line and for connecting electrical devices within the device shaft to control systems. Such connectors may be permanent or demountable. The hub may also comprise a coolant manifold with input and output connectors for connecting the coolant input to a coolant supply and the coolant output to waste or recirculating system. The hub may also form part of a handle configured to provide a firmer grip for a surgeon to handle the tissue ablation device.

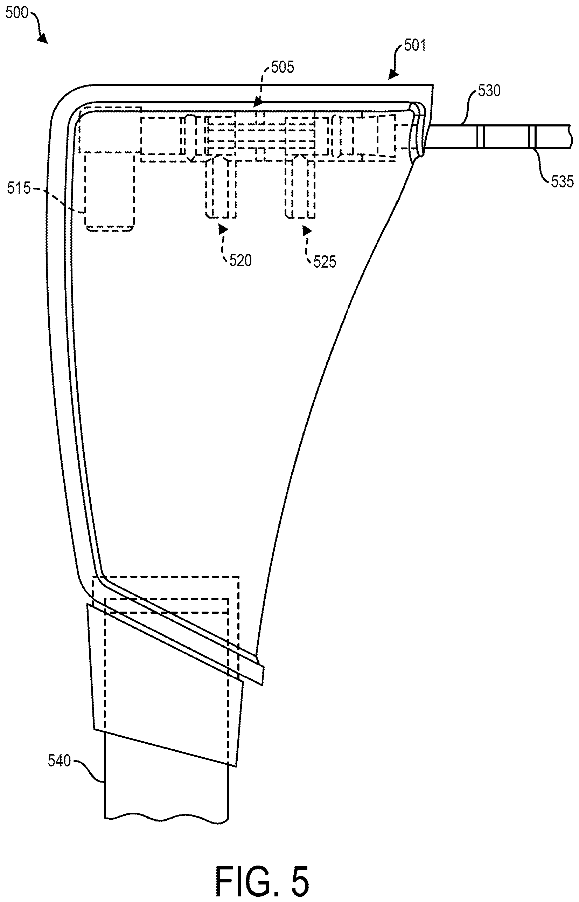

FIG. 5 is a side view of a microwave tissue ablation device according to one embodiment of the disclosure. The ablation device 500 includes a handle 501. The handle 501 houses a manifold 505.

The manifold 505 electrically connects the power source (not shown) and the tissue ablation probe 530 through the coaxial cable connector 515. The tissue ablation probe 530 includes markings 535 configured to inform surgeons of the depth of probe penetration during surgery,

The manifold 505 also fluidically connects the coolant source (not shown) and the tissue ablation probe 530. The manifold 505 includes a coolant inlet 520 and a coolant outlet 525. The coolant inlet 520 is fluidically connected to the coolant inflow conduit and the coolant outlet 525 is fluidically connected to the coolant outflow conduit.

The tissue ablation device 500 further includes tubular housing 540 that houses the electric wires and fluid tubes.

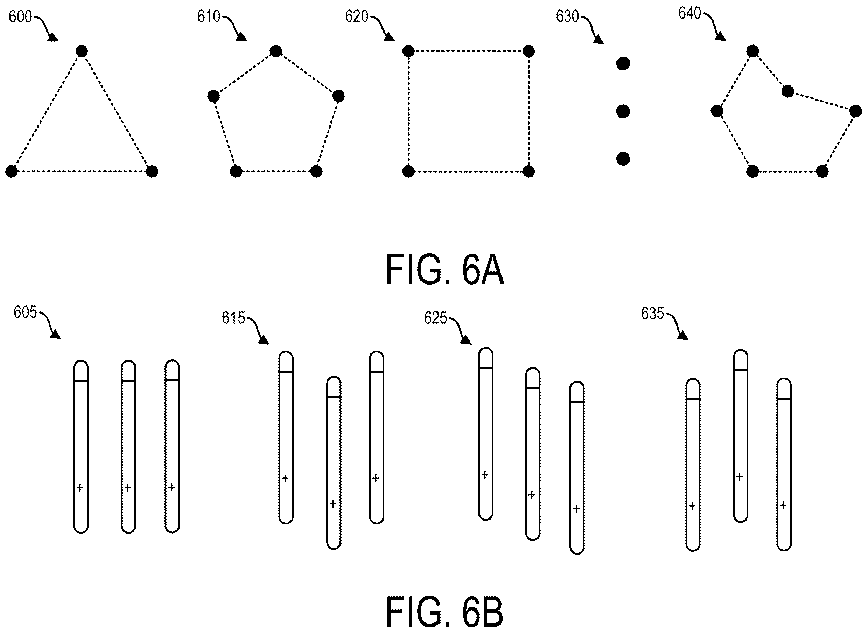

As discussed elsewhere herein, a plurality of ablation devices, such as tissue ablation device 300, can be used simultaneously to perform ablation processes. Such ablation devices may be arranged in a variety of ways. FIG. 6A shows a plain view of a plurality of microwave ablation needle configurations. In an example, microwave ablation devices can be positioned equidistant from each other, such as in arrangement, 600. Needles can be arranged in a regular polygon formation, such as in arrangements 600, 610, and 620. Positioning the ablation devices equidistant from one another may advantageously provide an approximately symmetric net ablation volume formed by the plurality of ablation devices. Additionally, arranging the ablation devices in a regular polygon formation may provide an approximately spherical net ablation volume formed by the plurality of ablation devices. Alternatively, other arrangements, a plurality of devices arranged in a line such as in arrangement, 630, or in an irregular shape such as in arrangement, 640. Ablation devices can be arranged in a plurality of configurations to provide a desired ablation volume appropriate for a particular operation.

In addition, such devices can be inserted to the same or different penetration depths. FIG. 6B shows an elevation view of a plurality of ablation devices at different depth arrangements. Ablation devices, such as microwave tissue ablation device 300, can be inserted to particular depths, for example, as gauged by markings 311. In some configurations, the devices are inserted to approximately the same depth, such as in arrangement, 605. In other examples, devices can be inserted to different depths, such as in arrangements 615, 625, and 635. Similar to different plan arrangements, ablation devices can be arranged in a plurality of configurations to provide a desired ablation volume appropriate for a particular operation.

During operation involving one or more ablation devices, a console (e.g., 102) can actuate a pump (e.g., 148a) to cause a coolant to flow from a coolant source (e.g., 140) to each of the one or more ablation devices (e.g., 400). For each ablation device, coolant can flow through a coolant line (e.g., 114a), a coolant inlet (e.g., 520), a first cooling conduit (e.g., 448), a second cooling conduit (e.g., 460), and a coolant outlet (e.g., 525). In some examples, coolant line (e.g., 114a) provides a return path to receive fluid from the coolant outlet (e.g., 525), for example, in a recirculation system in which coolant is recirculated to the coolant source. In an embodiment, coolant can flow through such a flow path to provide cooling to the ablation device.

As described herein, a controller (e.g., 106), for example, within a console (e.g., 102) receiving the ablation device (e.g., 400) can be used to control the fluid flow through the ablation device as well as the microwave energy emitted from the ablation device.

Coolant can also act as a dielectric to couple microwave radiation emitted from a microwave antenna (e.g., 452) to surrounding tissue when the microwave ablation device is inserted in a patient. In an embodiment, coolant flows through the needle during a treatment ablation process at a treatment ablation flow rate. Coolant flowing through the needle at the treatment ablation flow rate can couple the microwave energy emitted from the needle to the tissue surrounding the needle and impact the penetration depth of the microwave energy into the tissue. Reducing the flow of coolant can reduce the coupling of the microwave energy to the surrounding tissue, resulting in a smaller ablation zone. Additionally or alternatively, reduced coolant flow can reduce the ability of the needle to draw heat away from tissue proximate the needle, resulting in more localized heating of tissue proximate to the needle compared to higher flow rates.

During an ablation procedure, power is provided to an ablation device such that the ablation device emits energy in order to ablate the tissue surrounding the ablation device. For example, an ablation power of 30-150 W may be applied to an ablation device in order to perform a treatment ablation process. In a specific example, 90 W of ablation power may be applied to an ablation device to perform a treatment ablation process.

It is understood that an ablation power level can be controlled in at least two different ways, by controlling the amplitude or by using duty cycling. For example, if it is desired to apply an average power level of 40 W, that may be accomplished by either setting the power level at 40 W, or by setting the power level at a higher value, 80 W, and implementing a 50% duty cycle. Both methods of controlling power level have been contemplated.

As discussed herein, the ablation energy emitted by the ablation device as a result of ablation power being applied thereto typically heats the surrounding medium. When the surrounding medium comprises tissue, the ablation device may kill the tissue by heating the tissue over time within a target area proximate the ablation device.

When heating tissue within the target area, water found within the cells of the tissue as well as water found outside the cells, such as in the extracellular matrix, may also be heated. As water within the target area is heated, the water may vaporize causing the production of steam within the target area. The rapid increase of temperature within the target area may generate a significant amount of steam within the target area, causing the production of steam pockets within the tissue. During some procedures, an excessive amount of steam may build up causing the steam pockets to burst. The bursting of steam pockets, commonly referred to as steam pops, can cause undue stress on the tissue as well as discomfort to a patient. Thus, it is preferred to reduce the presence of steam pockets, and consequently steam pops, in order to minimize discomfort for the patient. In addition, the presence of steam pockets may cause non-uniform ablation and/or lead to tissue discontinuities, which may prevent heat from effectively transferring through the target tissue. Thus, reducing the presence of steam pockets may also provide for more uniform ablation and decreases tissue discontinuities, which may result in more effective ablation.

One way to minimize steam pops in the present invention is to first apply a lower level of ablation power to the ablation device and then increase the amount of ablation power applied over time until a desired ablation power level is reached. For instance, an initial ablation power of 30-60 W may be applied to the ablation device before the power is thereafter increased to a full-power level. In an example, an initial ablation power of 40 W can be used. In other examples, an ablation procedure may start at other ablation power levels, such as between 0 W and 30 W, or over 60 W, depending on the antenna design and area of treatment. Initiating ablation at a lower power may heat up tissue at a slower pace, allowing steam formed during the ablation procedure to escape or be absorbed, thereby reducing the formation of steam pockets and consequentially steam pops in the target area.

In embodiments wherein the ablation procedure is initiated with an ablation power that is lower than a treatment ablation power, ablation power may be increased from the lower ablation power to the higher treatment ablation power. While the lower initial ablation power can provide benefits as described herein, using the higher treatment ablation power may reduce the duration and increase the efficacy of the ablation procedure when compared to the initial lower ablation power. Increasing the ablation power from the lower power level to the higher power level may be done in a variety of ways, such as via a singular step, multiple steps, or a linear increase over time.

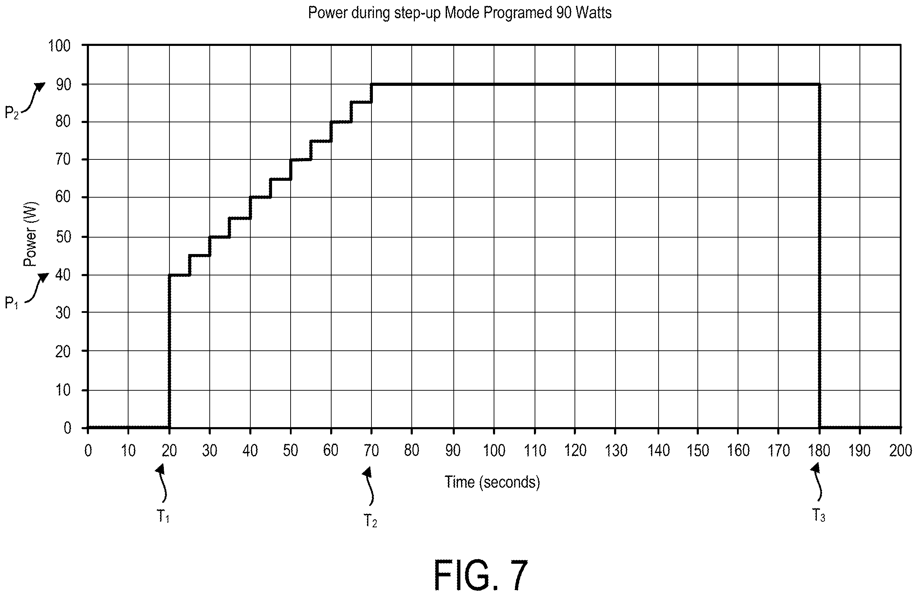

FIG. 7 shows an exemplarily ablation procedure using a Step-Up Mode of operation. The Step-Up Mode of operation may include a series of multiple steps used to increase from an initial, lower ablation power level (P₁) to a higher ablation power level (P₂). In preferred embodiments, the multiple steps comprise a plurality of distinct intermediate power levels wherein each of the intermediate power levels is between the lower ablation power level (P₁) and the higher ablation power level (P₂). In further preferred embodiments, each consecutive intermediate power level is greater than the previous intermediate power level. In some examples, each intermediate power level may be provided to an ablation device for a predetermined amount of time, for example, between approximately 1 second and 15 seconds. However amounts of time less than 1 second and more than 15 seconds have been contemplated. Power level P₂ is provided until time T₃, at which point ablation power is stopped or at least reduced.

FIG. 7 illustrates an example wherein the difference between each consecutive power level is approximately the same and each predetermined amount of time between the intermediate power levels is also approximately the same. In particular, in the illustrated example, the ablation power is increased from 40 W, an initial power level, to 90 W in even increments of 5 W every 5 seconds between a first time (T₁) and a second time (T₂). In some embodiments, the difference between T₁ and T₂ may be between 30 and 80 seconds, and potentially up to 2 to 3 minutes. In a particular example, ablation power is increased from an initial power to a desired power in 50 seconds, such as shown in FIG. 7. However, other values such as less than 30 seconds and more than 80 seconds have been contemplated. While longer amounts of time between power levels may result in more effective ablation, the use of longer amounts of time results in a longer overall procedure time, which may have disadvantages for patients. Also, the predetermined amount of time between intermediate power levels may not be the same throughout the procedure. For example, it may be advantageous to use longer amounts of time at lower ablation power levels and shorter amounts of time at higher ablation power levers, and vice versa.

Even though not shown with respect to FIG. 7, other step-up increments may be used. For example, increments between the intermediate power such as values between 1W and 10W may be used, however increments smaller than 1W and greater than 10W have been contemplated. In some examples, the intermediate power levels also do not need to be provided in even power increments and/or be provided for equal amounts of time. Furthermore, other functions may be used to increase the ablation power level from the lower ablation power level (P₁) to the higher ablation power level (P₂) such as a continuous or linear ramp up function or other functions known to one of ordinary skill in the art.

In some examples, the ablation procedure will increase from the first level of ablation power to a second level of ablation power in a single step after a predetermined amount of time. Alternatively, the ablation power may gradually step up from the first level of ablation power to the second level of ablation power using a plurality of distinct intermediate power levels.

In some examples, when a plurality of discrete power levels are applied between the first level of ablation power and the second level of ablation power, each increase in the power (step 820) is the same increase in magnitude (e.g., increasing by 5W each step). Similarly, in some examples, the predetermined amount of time at each power level is the same for each intermediate power level (e.g., 5s). However, in other examples, the amount of power need not increase by the same amount each time. Similarly, in some examples, the amount of time spent at each intermediate power level need not be the same for each intermediate power level. For example, it may be advantageous to use a sequence of a first amount of time at a first power level, followed by a shorter, second amount of time at a higher, second power level, and then a third amount of time at third power level. The first and third amounts of time may be the same or different, and the first and third power levels may also be the same or different. In particular, a sequence of a first amount of time at a first power level, followed by a shorter, second amount of time at a second, higher power level, and followed by a third amount of time at third power level, where the third amount of time is longer than the second amount of time and the third power level is higher than the second power level, may be used,

As discussed herein, in embodiments wherein the first level of ablation power is lower than the second level of ablation power, one or more distinct intermediate power levels between the first level of ablation power and the second level of ablation power can be applied, wherein each distinct intermediate power level is greater than the previous intermediate power level. However, additional power levels may be included. For example, when multiple ablation devices are used, ablation power may not always be provided to each ablation device simultaneously, for example, due to a duty cycling of the ablation power applied to different ablation device, This can result in periods of time wherein no ablation power is applied to an ablation device.

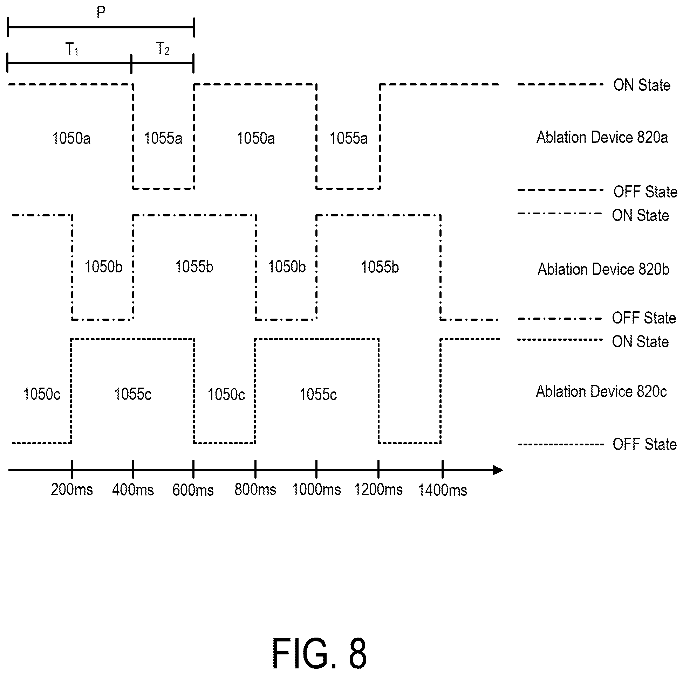

In such an example where ablation power is applied intermittently, such as via a duty cycle, ablation power applied to each ablation device may still be ramped over time. In some instances, the ablation system may provide each intermediate power level for a predetermined amount of time, but the power level may be subject to a duty cycle over the predetermined amount of time. For example, with respect to FIG. 7, power level P1 is applied for approximately 5 seconds before the power level is increased to an intermediate power level. During that 5 seconds, in some embodiments, the power applied to the ablation device may be subject to a duty cycle and include one or more periods of time during which the ablation device is in an OFF state. For example, duty cycling of a plurality of ablation devices is described in FIG. 8.

FIG. 8 shows a plot of ablation power provided to a plurality of ablation devices, FIG. 8 shows an exemplary embodiment of using duty cycles. With respect to FIG. 8, ablation signals provided to each ablation device 820a-c, have the same period (P), first amount of time (T₁), and second amount of time (T₂). As shown, in the illustrated example, T₂ is approximately twice the duration of T₁. More specifically, in the illustrated example; T₁ is approximately 200 ms and T₂ is approximately 400 ms. Further, the first amount of time for each ablation device is temporally offset from one another. More specifically, the first amount of time for a given ablation device operation is temporally offset by 200 ms from the first amount of time for the other ablation devices.

With regard to FIG. 8, the first illustrated 200 ms represents the ablation state wherein ablation devices 820a and 820b are in an ON state and ablation device 820c is in an OFF state, The time between 200-400 ms of FIG. 8 represents the ablation state wherein ablation devices 820a and 820b are in an ON state and ablation device 820b is in an OFF state. The time between 400-600 ms of FIG. 8 represents the ablation state wherein ablation devices 820b and 820c are in an ON state and ablation device 820a is in an OFF state. As can be seen, the pattern repeats by reverting back to the ablation state for the time between 600-900 ms of FIG. 8 and continues to repeat.

FIG. 8 shows that the three ablation devices (e.g, ablation devices 820a-c) cycle between states every 600 ms, In some embodiments, the cycle may take more or less time than 600 ms. For example, the ablation states may be cycled through quicker such that it takes less than 600 ms to cycle between the states (e.g., 200 ms to 600 ms). Alternatively, the ablation states may be cycled through slower such that it takes more than 600 ms to cycle between the states (e.g., 600 ms to 5 seconds). Additionally, times faster than 200 ms and slower than 5 seconds have been contemplated.

As shown in FIG. 8, the ablation power can be provided to a plurality of ablation devices having duty cycles temporally offset from each other. As used herein, temporal offset between duty cycles describes a difference in time between the transition between ON and OFF states of a duty cycle. For example, as shown in FIG. 8, the duty cycle associated with ablation device 820a is temporally offset from the duty cycle associated with ablation device 820b by approximately 200 ms. In some embodiments, the temporal offset between duty cycles is between approximately 50 ms and approximately 500 ms. In some embodiments, the temporal offset between duty cycles is between approximately 100 ms and approximately 300 ms. In other examples, temporal offsets shorter than 50 ms and longer than 500 ms can be used.

In embodiments, duty cycles associated with each ablation device have a similar period. In some such examples, the temporal offset between successive duty cycles can be approximately equal to the period divided by the number of ablation devices. For example, with respect to FIG. 8, the duty cycle period associated with each of the three ablation devices (820a-c) is approximately 600 ms, and the duty cycles are temporally offset between approximately 200 ms.

In some examples, the duty cycle itself may be implemented based on a variety of factors, including, for example, the number of ablation devices to be used, the maximum number of ablation devices that can be powered simultaneously, or the like. For instance, in examples in which three ablation devices are used and it is possible to power two ablation devices simultaneously, a 2/3 duty cycle (two parts on, one part off) may be implemented. Each ablation device can be provided with duty cycled ablation power, wherein the duty cycles for each ablation device are temporally offset from one another.

While FIG. 8 generally shows each ablation device having equal but temporally offset duty cycles, in alternative embodiments, various duty cycles need not be similar among ablation devices. In some embodiments, one or more of the ablation devices may operate according to different duty cycles, for example, having different amounts of time in the ON operational state (T₁) or in the OFF operational state (T₂). Ablation devices may also have the same or similar duty cycles, but have different periods (P) associated with the duty cycles thereof.

Additionally or alternatively, while FIG. 8 generally shows cycling between various ablation states, the pattern of changing between ablation states need not be cyclical. For example, in some embodiments, manual switching between different ablation states in any order can be performed manually by a physician, for example, via a user interface. In general, for systems with one or more ablation devices, any ablation states or order of applying such ablation states are possible.

Accordingly, when referring herein to providing power at a power level (e.g., an intermediate power level) for a predetermined amount of time, this does not preclude subjecting the applied power to a duty cycle (therefore having some portions of time in which no power is applied) or such power having some other time-varying signal characteristic (e.g., an AC square wave) having a variable amplitude over that predetermined amount of time.

In some embodiments, there may therefore be periods of time during one or more consecutive intermediate power levels wherein no ablation power is applied to the ablation device. In particular, such embodiments may be implemented when multiple ablation devices are used simultaneously and cycled between a plurality of ablation states, such as due to duty cycling. In some examples, periods of time in which no ablation power is provided to a particular ablation device (e.g., during an OFF state of a duty cycle) can, but need not, align with the beginning or ending of the application of a particular intermediate power level. In some examples, a duty cycle can occur multiple times during the application of a single intermediate ablation power, once during the application of a single intermediate ablation power, or once over the application of a plurality of intermediate ablation powers.

Once the second ablation power level is provided at the second time, the second ablation power level may be provided until a third time, As shown in FIG. 7, the second ablation power level (P₂) of 90W is provided until the third time (T₃) at 180 seconds. In some embodiments, the third time may represent the end of the ablation procedure. The third time may be a predetermined time, such as 30 seconds to 3 minutes after the initiation of the ablation procedure or 900 seconds after reaching the second level of ablation power at the second time.

Similar to that described above with respect to the intermediate power levels, delivering power at the second level of ablation power can be subject to a duty cycle and/or may include a signal having a changing amplitude over time (such as an AC square wave), potentially resulting in periods of time wherein no ablation power is applied to a particular ablation device or less than a peak power level is applied to the ablation device. Thus, while applying the second ablation power level over a predetermined amount of time, a constant magnitude of power need not be continuously provided between the second time (T₂) and the third time (T₃), for example, when multiple ablation devices are used and cycled between a plurality of states, as discussed herein.

Additionally or alternatively, the amount of time may be determined based on whether or not an energy goal has been met. For example, in an embodiment, the ablation device may continue to provide the second ablation power level until the energy delivered to the tissue reaches an energy delivery goal. In some examples, determining whether or not the energy goal is met may comprise calculating the amount of energy delivered from an ablation device, for example, by integrating a power vs. time curve. The integral of power vs. time (or an approximation thereof) provides a measure of the total energy delivered Thus, in some examples, such information can be monitored, for example, via the controller, to determine a total energy delivered from an ablation device during the ablation process.

In some examples, the controller can be configured to monitor delivered power and/or the reflected power of an ablation device. For example, in some cases due to a variety of factors, some of the power delivered to the ablation device can be reflected back within the ablation device. Such factors may include ablation signal factors (e.g., power level, frequency, etc.), composition of the target area (e.g., tissue type, water content, etc.), type of ablation device used, proximity to other media (e.g., bones, air pockets, additional ablation devices, auxiliary devices), or the like. For example, in some examples, poor coupling of the ablation device and the surrounding tissue can lead to power reflection within the ablation device, such as within a microwave ablation needle.

The ablation system may be able to determine the delivered power and/or the reflected power of the ablation device, such as by receiving information regarding the delivered power and/or the reflected power. In some embodiments, the ablation system may comprise a directional coupler, which can be used to measure received power as well as provide power. Such directional couplers can be used to measure power emitted from an ablation device, which can be compared to the provided power to the ablation device. Additionally or alternatively, other devices may also be used to determine delivered power and/or received power such as additional directional couplers on additional ablation devices or an antenna designated to receive power. When using a directional coupler or similar devices, reflected power may be determined by monitoring the received power (e.g., S₁₁ of a directional coupler). Additionally or alternatively, an ablation device may be used to determine an amount of power delivered, by one or more other ablation devices by monitoring the received power (e.g., S₂₁ or S₃₁ of a directional coupler, etc.). In preferred embodiments, an ablation device not supplying ablation power at a given time, such as during an off period of a duty cycle as described herein, can be used to determine the amount of delivered power.

In some embodiments, monitoring delivered power may be used to determine reflected power. In some embodiments, it is assumed that any power not received by a second device (e.g., a different ablation device) is power reflected by the ablation device and is power not emitted into the target area. However, the distance between the two devices may result in the power signal attenuating through the tissue. The attenuation may result in the received power by the second device not being the total delivered power, but rather slightly less. In some embodiments, the delivered power may be calculated using the received power, such as by calculating the attenuation using known properties of the tissue within the target area and the distance between the ablation device and the second device. Additionally, monitoring the reflected power may be used to determine the delivered power. In some embodiments, it is assumed that a power not reflected may be used to determine delivered power.

The reflected power and/or delivered power can be used to determine an efficiency of the ablation procedure, such as the ratio of delivered power to reflected power, the ratio of delivered power to provided power, or the ratio of the reflected power to provided power. In some examples, the calculated efficiency may be displayed to a user (e.g., a physician) on a graphic user interface,

In some embodiments, determined delivered power over time can be used to calculate an amount of energy delivered from an ablation device, compensating for the reflected power not directed to the tissue.

The ablation system may receive inputs regarding ablation parameters, such as the target area dimensions, the positioning of the one or more ablation devices, a prescribed ablation time, and/or a prescribed amount of energy. The ablation system may receive inputs regarding the diameter of the target area (e.g., 6.5 cm) and the arrangement of the ablation devices (e.g., arrangements shown in FIG. 6A and 6B). In some embodiments, the ablation system will calculate and recommend one or more ablation parameters, such as an ablation power level, a total energy amount, and/or an ablation duration for the ablation procedure, for example, based on a selected tissue or region for performing an ablation procedure. Such parameters can be determined, for example, using one or more equations and/or lookup tables stored in memory in response to one or more inputs provided by a user.

The ablation system may also recommend ramp up parameters as part of the ablation procedure parameters. The ramp up parameters may comprise the first level of ablation power, the second level of ablation power, the first time, and/or the second time as described with respect to FIG. 7 and 8. In some embodiments, the default ramp up parameters, such as the parameters shown in FIG. 7, may be used unless adjusted by a user. Alternatively, the ramp up parameters may be dependent on the ablation procedure parameters, such as the target area size, the number of ablation devices, the first level of ablation power, the second level of ablation power, the total energy amount to be delivered, the ablation duration and/or the like.

In some embodiments, a user may provide one or more inputs to interface with the ablation system. A user can provide an input to increase or decrease an amount of power being applied to an ablation device. Additionally or alternatively, a user can provide an input to increase or decrease an amount of time associated with an ablation, such as an amount of time to dwell at an intermediate power level and/or an amount of time to provide an ablation power (e.g., a second level of ablation power). A user can provide an input to stop applying ablation power, for example, to shut down the system.

Similarly, a user can provide one or more inputs at the start of an ablation process, such as providing a first ablation power level, a second ablation power level, a number of intermediate ablation power levels, an incremental change between consecutive intermediate ablation power levels, an amount of time to provide each intermediate ablation power level, and/or an amount of time to provide the second ablation power level.

Various non-limiting examples have been described. These and others are within the scope of the following claims. Additionally, while the invention is susceptible to various modifications and alternative forms, some specific embodiments thereof are shown by way of example in the drawings. The drawings may not be to scale.

## Claims

1. A tissue ablation system comprising:
a microwave ablation device (120a, 120b, 120c) configured to provide energy to a target area and configured to cause ablation by emission of microwave energy;
an ablation generator (138) configured to provide power to the ablation device; and
a controller (106) in communication with the ablation generator and being configured to:
cause the ablation generator to apply a first power level (P1) to the ablation device at a first time (T1); and
cause the ablation generator to apply a second power level (P2) to the ablation device at a second time (T2), the second power level being higher than the first power level and the second time being after the first time;
wherein the power provided by the ablation generator to the ablation device is adjustable from the first power level to the second power level between the first time and the second time, adjusting the power from the first power level to the second power level comprises causing the ablation generator to provide a plurality of distinct intermediate power levels corresponding to delivering a plurality of intermediate energy levels from the ablation device to the target area, and each of the intermediate power levels is between the first power level and the second power level, and each consecutive intermediate power level is greater than the previous intermediate power level, wherein each intermediate power level is between 1 W and 10 W greater than the previous intermediate power level and wherein a time between the first time and the second time is between 40 seconds and 100 seconds.

2. The tissue ablation system of claim 1, wherein the ablation generator is configured to provide power at each of the intermediate power levels for a predetermined amount of time.

3. The tissue ablation system of claim 1 or 2, wherein the plurality of distinct intermediate power levels are incremented using a step up function.

4. The tissue ablation system of any of the preceding claims, wherein:
the first power level is between 20 W and 60 W; and
the second power level is between 70 W and 150 W.

5. The tissue ablation system of any of the preceding claims, further comprising a coolant system for the ablation device, the coolant system comprising:
a coolant chamber, the coolant chamber surrounding a distal portion of the ablation device;
a coolant inlet, the coolant inlet configured to provide a coolant to the coolant chamber;
a coolant outlet, the coolant outlet configured to carry the coolant away from the coolant chamber; and
one or more pumps configured to supply the coolant to the coolant chamber.

6. The tissue ablation system of any of the preceding claims, further comprising:
a display configured to provide information regarding an ablation procedure; and
a user interface;
wherein the controller is in communication with the display and the user interface, and
is further configured to:
receive inputs from the user interface, the received inputs comprising at least one of: a total ablation time, a total ablation energy, and a dimension for an ablation zone;
determine ablation procedure parameters based on the received inputs; and
cause the ablation generator to apply ablation power to the ablation device based on the ablation procedure parameters.

7. The tissue ablation system of claim 6, wherein the user interface is further configured to receive inputs regarding the ablation procedure parameters during the ablation procedure.

8. The tissue ablation system of claim 7, wherein the received inputs during the ablation procedure comprise at least one of:
an input to increase the total ablation time, and an input to terminate the ablation procedure.

9. The tissue ablation system of claim 7, wherein the received inputs during the ablation procedure comprise an input to adjust the power level of the ablation power.

10. The tissue ablation system of claim 6, wherein the display is further configured to display at least one of:
a duration of the ablation procedure, a percent of total energy delivered, a current power level of the ablation power, and a power level of the ablation energy over time.

11. The tissue ablation system of any of the preceding claims, further comprising:
a second ablation device configured to provide energy to the target area;
the ablation generator configured to provide power to the second ablation device; and
the controller further configured to:
cause the ablation generator to apply a third power level to the second ablation device at a third time, the third power level corresponding to delivering a third level of energy from the second ablation device to the target area; and
cause the ablation generator to apply a fourth power level to the ablation device at a fourth time, the fourth power level corresponding to delivering a fourth level of energy from the second ablation device to the target area, wherein the fourth power level being higher than the third power level and the fourth time being after the third time;
wherein the power provided by the ablation generator to the second ablation device is adjusted from the third power level to the fourth power level between the third time and the fourth time, adjusting the power from the third power level to the fourth power level comprises causing the ablation generator to provide a plurality of distinct second ablation device intermediate power levels corresponding to delivering a plurality of second ablation device intermediate energy levels from the second ablation device to the target area, wherein each of the second ablation device intermediate power levels is between the third power level and the fourth power level, and each consecutive second ablation device intermediate power level is greater than the previous second ablation device intermediate power level; and
wherein the ablation generator provides power at each of the intermediate power levels and second ablation device intermediate power levels for a predetermined amount of time.

12. The tissue ablation system of claim 11, wherein the first power level is the same as the third power level and the second power level is the same as the fourth power level.

13. The tissue ablation system of claim 11, wherein the first time and third time are approximately the same; and
the second time and fourth time are approximately the same.

## Patentansprüche

1. Gewebeablationssystem, mit:
einer Mikrowellenablationsvorrichtung (120a, 120b, 120c), die dafür konfiguriert ist, einem Zielbereich Energie zuzuführen und eine Ablation durch Emission von Mikrowellenenergie zu veranlassen;
einem Ablationsgenerator (138), der dafür konfiguriert ist, die Ablationsvorrichtung mit Leistung zu versorgen; und
einer Steuereinheit (106), die mit dem Ablationsgenerator kommuniziert und dafür konfiguriert ist:
den Ablationsgenerator zu veranlassen, der Ablationsvorrichtung zu einem ersten Zeitpunkt (T1) einen ersten Leistungspegel (P1) zuzuführen; und
den Ablationsgenerator zu veranlassen, der Ablationsvorrichtung zu einem zweiten Zeitpunkt (T2) einen zweiten Leistungspegel (P2) zuzuführen,
wobei der zweite Leistungspegel höher ist als der erste Leistungspegel, und
der zweite Zeitpunkt nach dem ersten Zeitpunkt liegt,
wobei die durch den Ablationsgenerator der Ablationsvorrichtung zugeführte Leistung zwischen dem ersten Zeitpunkt und dem zweiten Zeitpunkt vom ersten Leistungspegel bis zum zweiten Leistungspegel einstellbar ist, wobei das Einstellen der Leistung vom ersten Leistungspegel bis zum zweiten Leistungspegel aufweist: Veranlassen, dass der Ablationsgenerator eine Vielzahl von unterschiedlichen Zwischenleistungspegeln bereitstellt, die der Zufuhr einer Vielzahl von Zwischenenergiepegeln von der Ablationsvorrichtung an den Zielbereich entsprechen, und wobei jeder der Zwischenleistungspegel zwischen dem ersten Leistungspegel und dem zweiten Leistungspegel liegt, und wobei jeder nachfolgende Zwischenleistungspegel größer ist als der vorherige Zwischenleistungspegel, wobei jeder Zwischenleistungspegel zwischen 1 W und 10 W größer ist als der vorherige Zwischenleistungspegel, und wobei eine Zeit zwischen dem ersten Zeitpunkt und dem zweiten Zeitpunkt zwischen 40 Sekunden und 100 Sekunden liegt.

2. Gewebeablationssystem nach Anspruch 1, wobei der Ablationsgenerator dafür konfiguriert ist, Leistung bei jedem der Zwischenleistungspegel für eine vorgegebene Zeitdauer bereitzustellen.

3. Gewebeablationssystem nach Anspruch 1 oder 2, wobei die Vielzahl unterschiedlicher Zwischenleistungspegel unter Verwendung einer Step-Up-Funktion erhöht werden.

4. Gewebeablationssystem nach einem der vorhergehenden Ansprüche, wobei:
der erste Leistungspegel zwischen 20 W und 60 W liegt; und
der zweite Leistungspegel zwischen 70 W und 150 W liegt.

5. Gewebeablationssystem nach einem der vorangehenden Ansprüche, ferner mit einem Kühlmittelsystem für die Ablationsvorrichtung, wobei das Kühlmittelsystem aufweist:
eine Kühlmittelkammer, wobei die Kühlmittelkammer einen distalen Abschnitt der Ablationsvorrichtung umgibt;
einen Kühlmitteleinlass, wobei der Kühlmitteleinlass dafür konfiguriert ist, der Kühlmittelkammer ein Kühlmittel zuzuführen;
einen Kühlmittelauslass, wobei der Kühlmittelauslass dafür konfiguriert ist, das Kühlmittel von der Kühlmittelkammer wegzutransportieren; und
eine oder mehrere Pumpen, die dafür konfiguriert sind, der Kühlmittelkammer das Kühlmittel zuzuführen.

6. Gewebeablationssystem nach einem der vorangehenden Ansprüche, ferner mit:
einer Anzeige, die dafür konfiguriert ist, Information über eine Ablationsprozedur bereitzustellen; und
einer Benutzerschnittstelle,
wobei die Steuereinheit mit der Anzeige und der Benutzerschnittstelle kommuniziert und ferner dafür konfiguriert ist:
Eingaben von der Benutzerschnittstelle zu empfangen, wobei die empfangenen Eingaben mindestens eine der folgenden Eingaben aufweisen: eine Gesamtablationszeit, eine Gesamtablationsenergie und eine Abmessung für eine Ablationszone;
Ablationsprozedurparameter basierend auf den empfangenen Eingaben zu bestimmen; und
den Ablationsgenerator zu veranlassen, basierend auf den Ablationsprozedurparametern der Ablationsvorrichtung Ablationsleistung zuzuführen.

7. Gewebeablationssystem nach Anspruch 6,
wobei die Benutzerschnittstelle ferner dafür konfiguriert ist, während der Ablationsprozedur Eingaben bezüglich der Ablationsprozedurparameter zu empfangen.

8. Gewebeablationssystem nach Anspruch 7,
wobei die während der Ablationsprozedur empfangenen Eingaben mindestens eine der folgenden Eingaben aufweisen:
eine Eingabe zum Erhöhen der Gesamtablationszeit und eine Eingabe zum Beenden der Ablationsprozedur.

9. Gewebeablationssystem nach Anspruch 7,
wobei die während der Ablationsprozedur empfangenen Eingaben eine Eingabe zum Einstellen des Leistungspegels der Ablationsleistung aufweisen.

10. Gewebeablationssystem nach Anspruch 6, wobei die Anzeige ferner dafür konfiguriert ist, mindestens eine der folgenden Informationen anzuzeigen:
eine Dauer der Ablationsprozedur, einen Prozentsatz der abgegebenen Gesamtenergie, einen aktuellen Leistungspegel der Ablationsleistung und einen Leistungspegel der Ablationsenergie über die Zeit.

11. Gewebeablationssystem nach einem der vorangehenden Ansprüche, ferner aufweisend:
eine zweite Ablationsvorrichtung, die dafür konfiguriert ist, dem Zielbereich Energie zuzuführen;
den Ablationsgenerator, der dafür konfiguriert ist, der zweiten Ablationsvorrichtung Leistung zuzuführen; und
die Steuereinheit, die ferner dafür konfiguriert ist:
den Ablationsgenerator zu veranlassen, zu einem dritten Zeitpunkt der zweiten Ablationsvorrichtung einen dritten Leistungspegel zuzuführen, wobei der dritte Leistungspegel der Abgabe eines dritten Energiepegels von der zweiten Ablationsvorrichtung an den Zielbereich entspricht; und
den Ablationsgenerator zu veranlassen, der Ablationsvorrichtung zu einem vierten Zeitpunkt einen vierten Leistungspegel zuzuführen, wobei der vierte Leistungspegel der Abgabe eines vierten Energiepegels von der zweiten Ablationsvorrichtung an den Zielbereich entspricht,
wobei der vierte Leistungspegel höher ist als der dritte Leistungspegel und der vierte Zeitpunkt nach dem dritten Zeitpunkt liegt,
wobei die der zweiten Ablationsvorrichtung durch den Ablationsgenerator zugeführte Leistung zwischen dem dritten und dem vierten Zeitpunkt vom dritten Leistungspegel auf den vierten Leistungspegel eingestellt wird, wobei das Einstellen der Leistung vom dritten Leistungspegel auf den vierten Leistungspegel beinhaltet: Veranlassen, dass der Ablationsgenerator eine Vielzahl von unterschiedlichen zweiten Ablationsvorrichtungs-Zwischenleistungspegeln bereitstellt, die der Abgabe einer Vielzahl von zweiten Ablationsvorrichtungs-Zwischenenergiepegeln von der zweiten Ablationsvorrichtung an den Zielbereich entsprechen, wobei jeder der zweiten Ablationsvorrichtungs-Zwischenleistungspegel zwischen dem dritten Leistungspegel und dem vierten Leistungspegel liegt und jeder nachfolgende zweite Ablationsvorrichtungs-Zwischenleistungspegel größer ist als der vorherige zweite Ablationsvorrichtungs-Zwischenleistungspegel, und
wobei der Ablationsgenerator Leistung bei jedem der Zwischenleistungspegel und zweiten Ablationsvorrichtungs-Zwischenleistungspegel für eine vorgegebene Zeitdauer bereitstellt.

12. Gewebeablationssystem nach Anspruch 11, wobei
der erste Leistungspegel gleich dem dritten Leistungspegel ist und der zweite Leistungspegel gleich dem vierten Leistungspegel ist.

13. Gewebeablationssystem nach Anspruch 11, wobei der erste Zeitpunkt und der dritte Zeitpunkt ungefähr gleich sind und der zweite Zeitpunkt und der vierte Zeitpunkt ungefähr gleich sind.

## Revendications

1. Système d'ablation de tissu comprenant :
un dispositif d'ablation par micro-ondes (120a, 120b, 120c) configuré pour fournir de l'énergie à une zone cible et configuré pour entraîner l'ablation par émission d'énergie micro-onde ;
un générateur d'ablation (138) configuré pour fournir de la puissance au dispositif d'ablation ; et
un dispositif de commande (106) en communication avec le générateur d'ablation et étant configuré pour :
amener le générateur d'ablation à appliquer un premier niveau de puissance (P1) au dispositif d'ablation à un premier moment (T1) ; et
amener le générateur d'ablation à appliquer un deuxième niveau de puissance (P2) au dispositif d'ablation à un deuxième moment (T2), le deuxième niveau de puissance étant plus élevé que le premier niveau de puissance et le deuxième moment survenant après le premier moment ;
dans lequel la puissance fournie par le générateur d'ablation au dispositif d'ablation est ajustable du premier niveau de puissance au deuxième niveau de puissance entre le premier moment et le deuxième moment, l'ajustement de la puissance du premier niveau de puissance au deuxième niveau de puissance comprend le fait d'amener le générateur d'ablation à fournir une pluralité de niveaux de puissance intermédiaires distincts correspondant à la délivrance d'une pluralité de niveaux d'énergie intermédiaires du dispositif d'ablation à la zone cible, et chacun des niveaux de puissance intermédiaires est entre le premier niveau de puissance et le deuxième niveau de puissance, et chaque niveau de puissance intermédiaire consécutif est supérieur au niveau de puissance intermédiaire précédent, dans lequel chaque niveau de puissance intermédiaire est entre 1 W et 10 W supérieur au niveau de puissance intermédiaire précédent et dans lequel un moment entre le premier moment et le deuxième moment est compris entre 40 secondes et 100 secondes.

2. Système d'ablation de tissu selon la revendication 1, dans lequel le générateur d'ablation est configuré pour fournir de la puissance à chacun des niveaux de puissance intermédiaires pendant une quantité de temps prédéterminée.

3. Système d'ablation de tissu selon la revendication 1 ou 2, dans lequel la pluralité de niveaux de puissance intermédiaires distincts sont incrémentés à l'aide d'une fonction de montée en charge.

4. Système d'ablation de tissu selon l'une quelconque des revendications précédentes, dans lequel :
le premier niveau de puissance est compris entre 20 W et 60 W ; et
le deuxième niveau de puissance est compris entre 70 W et 150 W.

5. Système d'ablation de tissu selon l'une quelconque des revendications précédentes, comprenant en outre un système de refroidissement pour le dispositif d'ablation, le système de refroidissement comprenant :
une chambre de refroidissement, la chambre de refroidissement entourant une portion distale du dispositif d'ablation ;
une entrée d'agent de refroidissement, l'entrée d'agent de refroidissement étant configurée pour fournir un agent de refroidissement à la chambre de refroidissement ;
une sortie d'agent de refroidissement, la sortie d'agent de refroidissement étant configurée pour évacuer l'agent de refroidissement de la chambre de refroidissement ; et
une ou plusieurs pompes configurées pour fournir l'agent de refroidissement à la chambre de refroidissement.

6. Système d'ablation de tissu selon l'une quelconque des revendications précédentes, comprenant en outre :
un affichage configuré pour fournir des informations concernant une procédure d'ablation ; et
une interface utilisateur ;
dans lequel le dispositif de commande est en communication avec l'affichage et l'interface utilisateur, et est en outre configuré pour :
recevoir des entrées de l'interface utilisateur, les entrées reçues comprenant au moins l'un parmi : un moment d'ablation totale, une énergie d'ablation totale et une dimension pour une zone d'ablation ;
déterminer des paramètres de procédure d'ablation sur la base des entrées reçues ; et
amener le générateur d'ablation à appliquer une puissance d'ablation au dispositif d'ablation sur la base des paramètres de procédure d'ablation.

7. Système d'ablation de tissu selon la revendication 6,
dans lequel l'interface utilisateur est en outre configurée pour recevoir des entrées concernant les paramètres de procédure d'ablation pendant la procédure d'ablation.

8. Système d'ablation de tissu selon la revendication 7,
dans lequel les entrées reçues pendant la procédure d'ablation comprennent au moins l'une parmi :
une entrée pour augmenter le temps d'ablation total et une entrée pour terminer la procédure d'ablation.

9. Système d'ablation de tissu selon la revendication 7,
dans lequel les entrées reçues pendant la procédure d'ablation comprennent une entrée pour ajuster le niveau de puissance de la puissance d'ablation.

10. Système d'ablation de tissu selon la revendication 6,
dans lequel l'affichage est en outre configuré pour afficher au moins l'un parmi :
une durée de la procédure d'ablation, un pourcentage d'énergie totale délivrée, un niveau de puissance actuel de la puissance d'ablation et un niveau de puissance de l'énergie d'ablation au fil du temps.

11. Système d'ablation de tissu selon l'une quelconque des revendications précédentes, comprenant en outre :
un deuxième dispositif d'ablation configuré pour fournir de l'énergie à la zone cible ;
le générateur d'ablation configuré pour fournir de la puissance au deuxième dispositif d'ablation ; et
le dispositif de commande configuré en outre pour :
amener le générateur d'ablation à appliquer un troisième niveau de puissance au deuxième dispositif d'ablation à un troisième moment, le troisième niveau de puissance correspondant à la délivrance d'un troisième niveau d'énergie du deuxième dispositif d'ablation à la zone cible ; et
amener le générateur d'ablation à appliquer un quatrième niveau de puissance au dispositif d'ablation à un quatrième moment, le quatrième niveau de puissance correspondant à la délivrance d'un quatrième niveau d'énergie du deuxième dispositif d'ablation à la zone cible, dans lequel le quatrième niveau de puissance est plus élevé que le troisième niveau de puissance et le quatrième moment survenant après le troisième moment ;
dans lequel la puissance fournie par le générateur d'ablation au deuxième dispositif d'ablation est ajustée du troisième niveau de puissance au quatrième niveau de puissance entre le troisième moment et le quatrième moment, l'ajustement de la puissance du troisième niveau de puissance au quatrième niveau de puissance comprend le fait d'amener le générateur d'ablation à fournir une pluralité de niveaux de puissance intermédiaires de deuxième dispositif d'ablation distincts correspondant à la délivrance d'une pluralité de niveaux d'énergie intermédiaires de deuxième dispositif d'ablation du deuxième dispositif d'ablation à la zone cible, dans lequel chacun des niveaux de puissance intermédiaires de deuxième dispositif d'ablation est entre le troisième niveau de puissance et le quatrième niveau de puissance, et chaque niveau de puissance intermédiaire de deuxième dispositif d'ablation consécutif est supérieur au niveau de puissance intermédiaire de deuxième dispositif d'ablation précédent ; et
dans lequel le générateur d'ablation fournit de la puissance à chacun des niveaux de puissance intermédiaires et des niveaux de puissance intermédiaires de deuxième dispositif d'ablation pendant une quantité de temps prédéterminée.

12. Système d'ablation de tissu selon la revendication 11,
dans lequel le premier niveau de puissance est le même que le troisième niveau de puissance et le deuxième niveau de puissance est le même que le quatrième niveau de puissance.

13. Système d'ablation de tissu selon la revendication 11,
dans lequel le premier moment et le troisième moment sont approximativement les mêmes ; et
le deuxième moment et le quatrième moment sont approximativement les mêmes.
